# EUROPEAN PATENT SPECIFICATION

(11) **EP 1 789 053 B1**
(45) Date of publication and mention of the grant of the patent: **13.06.2012**
(21) Application number: 05811901.7
(22) Date of filing: 30.08.2005
(51) Int. Cl.: A61K 31/522, A61P 17/02, A61P 1/04

(54) **METHOD OF WOUND HEALING USING A2B ADENOSINE RECEPTOR ANTAGONISTS**
VERFAHREN ZUR WUNDHEILUNG MITHILFE VON A2B-ADENOSINREZEPTOR-ANTAGONISTEN
PROCEDE DE CICATRISATION EN UTILISANT LES ANTAGONISTES DES RECEPTEURS A2B DE L'ADENSOINE

(30) Priority: 01.09.2004 US 606675 P
(43) Date of publication of application: 30.05.2007
(73) Proprietor: Gilead Sciences, Inc., Foster City, CA 94404 (US)
(72) Inventor: ZENG, Dewan, San Mateo, CA 94403 (US); BELARDINELLI, Luiz, Palo Alto, CA 94306 (US)
(74) Representative: Schnappauf, Georg
(86) International application number: PCT/US2005/030838
(87) International publication number: WO 2006/028810

(56) References cited:
- US-A1- 2003 229 106
- ROHOVSKY S AND D'AMORE P A: "Growth factors and angiogenesis in wound healing" GROWTH FACTORS WOUND HEALING, 1997, pages 8-26, XP002099410
- FEOKTISTOV I ET AL: "Adenosine A(2B) receptors" PHARMACOLOGICAL REVIEWS, WILLIAMS AND WILKINS INC., BALTIMORE, MD,, US, vol. 49, no. 4, 1997, pages 381-402, XP002113960 ISSN: 0031-6997
- FEOKTISTOV I ET AL: "Adenosine A2B receptors: a novel therapeutic target in asthma?" TRENDS IN PHARMACOLOGICAL SCIENCES, ELSEVIER, HAYWARTH, GB, vol. 19, no. 4, 1 April 1998 (1998-04-01), pages 148-153, XP004117815 ISSN: 0165-6147
- DATABASE MEDLINE [Online] US NATIONAL LIBRARY OF MEDICINE (NLM), BETHESDA, MD, US; May 2005 (2005-05), DESAI AVANI ET AL: "Adenosine A2A receptor stimulation increases angiogenesis by down-regulating production of the antiangiogenic matrix protein thrombospondin 1." XP002390017 Database accession no. NLM15673602 & MOLECULAR PHARMACOLOGY. MAY 2005, vol. 67, no. 5, May 2005 (2005-05), pages 1406-1413, ISSN: 0026-895X

## Description

### Field of the Invention

The present invention relates to A₂B adenosine receptor antagonists for use in methods of wound healing. The invention also relates to pharmaceutical compositions containing such compounds for use in augmenting wound healing.

### Background

Adenosine is a naturally occurring nucleoside, which exerts its biological effects by interacting with a family of adenosine receptors known as A₁, A_{2A}, A_{2B}, and A₃, all of which modulate important physiological processes. For example, A_{2A} adenosine receptors modulate coronary vasodilation, A_{2B} receptors have been implicated in mast cell activation, asthma, vasodilation, regulation of cell growth, intestinal function, and modulation of neurosecretion (See Adenosine A2B Receptors as Therapeutic Targets, Drug Dev.Res.45:198; Feoktistov et al., Trends Pharmacol.Sci. 19:148-153), and A₃ adenosine receptors modulate cell proliferation processes.

Adenosine A_{2B} receptors are ubiquitous, and regulate multiple biological activities. For example, adenosine binds to A_{2B} receptors on endothelial cells, thereby stimulating angiogenesis. Adenosine also regulates the growth of smooth muscle cell populations in blood vessels. Adenosine stimulates A_{2B} receptors on mast cells, thus modulating Type I hypersensitivity reactions. Adenosine also stimulates gastrosecretory activity by activation with A_{2B} in the intestine.

As discussed above, the binding of A_{2B} receptors stimulates angiogenesis by promoting the growth of endothelial cells. It has long been suggested that since such activity is necessary in healing wounds, agonists of the A_{2B} receptor would be useful in wound healing. Surprisingly, it has now been discovered that A_{2B} antagonists are also effective in wound healing applications.

Accordingly, it is desired to augment wound healing by administration of compounds that are potent A_{2B} antagonists (i.e., compounds that inhibit the A_{2B} adenosine receptor), fully or partially selective for the A_{2B} receptor.

### SUMMARY OF THE INVENTION

In one aspect, the invention provides the use of an A_{2B} receptor antagonist having the structure of Formula I or Formula II: 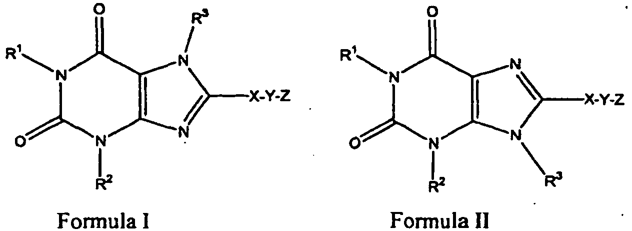 wherein:
R¹ and R² are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl, or optionally substituted alkynyl, with the proviso that when D is a covalent bond E cannot be alkoxy;
R³ is hydrogen, optionally substituted alkyl or optionally substituted cycloalkyl;
X is optionally substituted arylene or heteroarylene;
Y is a covalent bond or alkylene in which one carbon atom can be optionally replaced by -O-, -S-, or -NH-, and is optionally substituted by hydroxy, alkoxy, optionally substituted amino, or -COR, in which R is hydroxy, alkoxy or amino;
   with the proviso that when the optional substitution is hydroxy or amino said substitution cannot be present on a carbon atom adjacent to a heteroatom; and
Z is hydrogen, optionally substituted monocyclic aryl or optionally substituted monocyclic heteroaryl; with the proviso that
   (a) Z is hydrogen only when Y is a covalent bond and X is optionally substituted 1,4-pyrazolene attached to the purine ring by a carbon atom; and
   (b) when X is optionally substituted arylene, Z is an optionally substituted monocyclic heteroaryl other than optionally substituted imidazole,
for the preparation of a pharmaceutical composition for accelerating wound healing in a mammal.
Preferred embodiments of this aspect of the invention are set forth in claims 2 to 34. The A_{2B} receptor antagonist may be administered topically and may be administered directly to the wound.

The wound to be treated may be caused by mechanical, chemical or thermal means and may take the form of a contusion, incision or laceration. The wound can be the result of a surgical incision or may be associated with a disease or disorder, such as diabetes. In particular, the wound may be a diabetic ulcer.

In yet another aspect of the invention, pharmaceutical formulations for use in augmenting wound healing and suitable for topical delivery are provided, comprising a therapeutically effective amount of an A_{2B} receptor antagonist having the structure of the above Formula I or II , and at least one pharmaceutically acceptable carrier. In one embodiment the pharmaceutical composition may be an ointment, lotion, cream, microemulsion, gel, oil or solution. In another embodiment the pharmaceutical composition is suitable for systemic delivery.

The formulation may contain one or more additional active agents and/or additives such as solubilizers, skin permeation enhancers, opacifiers, preservatives (e.g., anti-oxidants), gelling agents, buffering agents, surfactants, emulsifiers, emollients, thickening agents, stabilizers, humectants, colorants and fragrance.

One preferred group of compounds of Formula I and II are those in which R¹ and R² are independently hydrogen, optionally substituted lower alkyl, or a group -D-E, in which D is a covalent bond or alkylene, and E is optionally substituted phenyl, optionally substituted cycloalkyl, optionally substituted alkenyl or optionally substituted alkynyl, particularly those in which R³ is hydrogen.

Within this group, a first class of compounds include those in which X is optionally substituted phenylene and Y is a covalent bond or lower alkylene in which one carbon atom can be optionally replaced by -O-, -S-, or -NH-. In one subgroup of this category, R¹ and R² are independently lower alkyl optionally substituted by cycloalkyl and in a still further subcategory, R and R² are n-propyl, Y is -OCH₂-, and Z is optionally substituted oxadiazole, particularly optionally substituted [1,2,4]-oxadiazol-3-yl, especially [1,2,4]-oxadiazol-3-yl substituted by optionally substituted phenyl or optionally substituted pyridyl.

A second class of compounds within this group include those in which X is optionally substituted pyrazolene. Within this class, a subclass can be defined wherein Y is a covalent bond, lower alkylene optionally substituted by hydroxy, alkoxy, optionally substituted amino, or -COR, in which R is hydroxy, alkoxy or amino; and Z is hydrogen, optionally substituted phenyl, optionally substituted oxadiazolyl, optionally substituted isoxazolyl, or optionally substituted pyridyl.

A specific subclass may be also be found wherein X is optionally substituted 1,4-pyrazolene and Z is optionally substituted phenyl. In some embodiments within this subclass, R¹ is lower alkyl optionally substituted by cycloalkyl, R² is hydrogen, and Y is -CH₂- or -CH(CH₃)-. In other embodiments within this subclass, R¹ and R² are independently methyl, ethyl, n-propyl, or cyclopropylmethyl, and Y is methylene or ethylene which may be optionally substituted by hydroxy, alkoxy, optionally substituted amino, or -COR, in which R is hydroxy, alkoxy or amino.

Another specific subclass may be found wherein Z is optionally substituted oxadiazole, Y is -CH₂- or -CH(CH₃)-, and R¹ is lower alkyl optionally substituted by cycloalkyl and R² is H, or R¹ and R² are independently lower alkyl optionally substituted by cycloalkyl. Still further specific subclasses can be defined where R¹ and R² are independently lower alkyl optionally substituted by cycloalkyl, and Y is tCH₂-, -CH(CH₃)- or a covalent bond, and Z is hydrogen, optionally substituted isoxazolyl, or pyridyl.

At present, the preferred compounds for use in the invention include, but are not limited to:

1-propyl-8-(1-{[3-(trifluoromethyl)phenyl]methyl}pyrazol-4-yl)-1,3,7-trihydropurine-2,6-dione;

1-propyl-8-[1-benzylpyrazol-4-yl]-1,3,7-trihydropurine-2,6-dione;

1-butyl-8-(1-{[3-fluorophenyl]methyl}pyrazol-4-yl)-1,3,7-trihydropurine-2,6-dione;

1-propyl-8-[1-(phenylethyl)pyrazol-4-yl]-1,3,7-trihydropurine-2,6-dione;

8-(1-{[5-(4-chlorophenyl)(1,2,4-oxadiazol-3-yl)]methyl}pyrazol-4-yl)-1-propyl-1,3,7-trihydropurine-2,6-dione;

8-(1-{[5-(4-chlorophenyl)(1,2,4-oxadiazol-3-yl)]methyl}pyrazol-4-yl)-1-butyl-1,3,7-trihydropurine-2,6-dione;

1,3-dipropyl-8-pyrazol-4-yl-1,3,7-trihydropurine-2,6-dione;

1-methyl-3-sec-butyl-8-pyrazol-4-yl-1,3,7-trihydropurine-2,6-dione;

1-cyclopropylmethyl-3-methyl-8-{1-[(3-trifluoromethylphenyl)methyl]pyrazol-4-yl}-1,3,7-trihydropurine-2,6-dione;

1,3-dimethyl-8-{1-[(3-fluorophenyl)methyl]pyrazol-4-yl}-1,3,7-trihydropurine-2,6-dione;

3-methyl-1-propyl-8-{1-[(3-trifluoromethylphenyl)methyl]pyrazol-4-yl}-1,3,7-trihydropurine-2,6-dione;

3-ethyl-1-propyl-8-{1-[(3-trifluoromethylphenyl)methyl]pyrazol-4-yl}-1,3,7-trihydropurine-2,6-dione;

1,3-dipropyl-8-(1-{[3-(trifluoromethyl)phenyl]methyl}pyrazol-4-yl)-1,3,7-trihydropurine-2,6-dione;

1,3-dipropyl-8-{1-[(3-fluorophenyl)methyl]pyrazol-4-yl}-1,3,7-trihydropurine-2,6-dione;

1-ethyl-3-methyl-8-{1-[(3-fluorophenyl)methyl]pyrazol-4-yl}-1,3,7-trihydropurine-2,6-dione;

1,3-dipropyl-8-{1-[(2-methoxyphenyl)methyl]pyrazol-4-yl}-1,3,7-trihydropurine-2,6-dione;

1,3-dipropyl-8-(1-{[3-(trifluoromethyl)phenyl]ethyl}pyrazol-4-yl)-1,3,7-trihydropurine-2,6-dione;

1,3-dipropyl-8-{1-[(4-carboxyphenyl)methyl]pyrazol-4-yl}-1,3,7-trihydropurine-2,6-dione;

2-[4-(2,6-dioxo-1,3-dipropyl-1,3,7-trihydropurin-8-yl)pyrazolyl]-2-phenylacetic acid;

8-{4-[5-(2-methoxyphenyl)-[1,2,4]oxadiazol-3-ylmethoxy]phenyl}-1,3-dipropyl-1,3,7-trihydropurine-2,6-dione;

8-{4-[5-(3-methoxyphenyl)-[1,2,4]oxadiazol-3-ylmethoxy]phenyl}-1,3-dipropyl-1,3,7-trihydropurine-2,6-dione;

8-{4-[5-(4-fluorophenyl)-[1,2,4]oxadiazol-3-ylmethoxy]phenyl}-1,3-dipropyl-1,3,7-trihydropurine-2,6-dione.

1-(cyclopropylmethyl)-8-[1-(2-pyridylmethyl)pyrazol-4-yl]-1,3,7-trihydropurine-2,6-dione;

1-n-butyl-8-[1-(6-trifluoromethylpyridin-3-ylmethyl)pyrazol-4-yl]-1,3,7-trihydropurine-2,6-dione;

8-(1-{[3-(4-chlorophenyl)(1,2,4-oxadiazol-5-yl)]methyl}pyrazol-4-yl)-1,3-dipropyl-1,3,7-trihydropurine-2,6-dione;

1,3-dipropyl-8-[1-({5-[4-(trifluoromethyl)phenyl]isoxazol-3-yl}methyl)pyrazol-4-yl]-1,3,7-trihydropurine-2,6-dione;

1,3-dipropyl-8-[1-(2-pyridylmethyl)pyrazol-4-yl]-1,3,7-trihydropurine-2,6-dione;

3-{[4-(2,6-dioxo-1,3-dipropyl-1,3,7-trihydropurin-8-yl)pyrazolyl]methyl}benzoic acid;

1,3-dipropyl-8-(1-{[6-(trifluoromethyl)(3-pyridyl)]methyl}pyrazol-4-yl)-1,3,7-trihydropurine-2,6-dione;

1,3-dipropyl-8-{1-[(3-(1H-1,2,3,4-tetrazol-5-yl)phenyl)methyl]pyrazol-4-yl}-1,3,7-trihydropurine-2,6-dione;

6-{[4-(2,6-dioxo-1,3-propyl-1,3,7-trihydropurin-8-yl)pyrazolyl]methyl}pyridine-2-carboxylic acid;

3-ethyl-1-propyl-8-[1-(2-pyridylmethyl)pyrazol-4-yl]-1,3,7-trihydropurine-2,6-dione;

8-(1-{[5-(4-chlorophenyl)isoxazol-3-yl]methyl}pyrazol-4-yl)-3-ethyl-1-propyl-1,3,7-trihydropurine-2,6-dione;

8-(1-{[3-(4-chlorophenyl)(1,2,4-oxadiazol-5-yl)]methyl}pyrazol-4-yl)-3-ethyl-1-propyl-1,3,7-trihydropurine-2,6-dione;

3-ethyl-1-propyl-8-(1-{[6-(trifluoromethyl)(3-pyridyl)]methyl}pyrazol-4-yl)-1,3,7-trihydropurine-2,6-dione;

1-(cyclopropylmethyl)-3-ethyl-8-(1-{[6-(trifluoromethyl)(3-pyridyl)]methyl}pyrazol-4-yl)-1,3,7-trihydropurine-2,6-dione; and

3-ethyl-1-(2-methylpropyl)-8-(1-{[6-(trifluoromethyl)(3-pyridyl)]methyl}pyrazol-4-yl)-1,3,7-trihydropurine-2,6-dione.

### DETAILED DISCRIPTION OF THE INVENTION

### Definitions and General Parameters

As used in the present specification, the following words and phrases are generally intended to have the meanings as set forth below, except to the extent that the context in which they are used indicates otherwise.

The term "alkyl" refers to a monoradical branched or unbranched saturated hydrocarbon chain having 1, 2, 3, 4, 5, 6, 7, 8, 9, 10, 11, 12, 13, 14, 15, 16, 17, 18, 19 or 20 carbon atoms. This term is exemplified by groups such as methyl, ethyl, n-propyl, iso-propyl, n-butyl, iso-butyl, t-butyl, n-hexyl, n-decyl, tetradecyl.

The term "substituted alkyl" refers to:
1) an alkyl group as defined above, having 1, 2, 3, 4 or 5 substituents, preferably 1 to 3 substituents, selected from the group consisting of alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino,
   nitro, -SO-alkyl, -SO-aryl, -SO-heteroaryl, -SO₂-alkyl, -SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0,1 or 2; or
2) an alkyl group as defined above that is interrupted by 1-10 atoms independently chosen from oxygen, sulfur and NRₐ-, where Rₐ is chosen from hydrogen, alkyl, cycloalkyl, alkenyl, cycloalkenyl, alkynyl, aryl, heteroaryl and heterocyclyl. All substituents may be optionally further substituted by alkyl, alkoxy, halogen, CF₃, amino, substituted amino, cyano, or -S(O)ₙR, in which R is alkyl, aryl, or heteroaryl and n is 0, 1 or 2; or
3) an alkyl group as defined above that has both 1, 2, 3, 4 or 5 substituents as defined above and is also interrupted by 1-10 atoms as defined above.

The term "lower alkyl" refers to a monoradical branched or unbranched saturated hydrocarbon chain having 1, 2, 3, 4, 5, or 6 carbon atoms. This term is exemplified by groups such as methyl, ethyl, n-propyl iso-propyl, n-butyl, iso-butyl, t-butyl, n-hexyl.

The term "substituted lower alkyl" refers to lower alkyl as defined above having 1 to 5 substituents, preferably 1, 2, or 3 substituents, as defined for substituted alkyl, or a lower alkyl group as defined above that is interrupted by 1, 2, 3, 4, or 5 atoms as defined for substituted alkyl, or a lower alkyl group as defined above that has both 1, 2, 3, 4 or 5 substituents as defined above and is also interrupted by 1, 2, 3, 4, or 5 atoms as defined above.

The term "alkylene" refers to a diradical of a branched or unbranched saturated hydrocarbon chain, having 1, 2, 3, 4, 5, 6, 7, 8, 9, 10, 11,12, 13,14, 15,16,17, 18, 19 or 20 carbon atoms, preferably 1-10 carbon atoms, more preferably 1, 2, 3, 4, 5 or 6 carbon atoms. This term is exemplified by groups such as methylene (-CH₂-), ethylene (-CH₂CH₂-), the propylene isomers (e.g., -CH₂CH₂CH₂- and -CH(CH₃)CH₂-) and the like.

The term "lower alkylene" refers to a diradical of a branched or unbranched saturated hydrocarbon chain, preferably having from 1, 2, 3, 4, 5, or 6 carbon atoms.

The term "lower alkylene" refers to a diradical of a branched or unbranched saturated hydrocarbon chain, preferably having from 1, 2, 3, 4, 5, or 6 carbon atoms.

The term "substituted alkylene" refers to:
(1) an alkylene group as defined above having 1, 2, 3, 4, or 5 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, -SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0,1 or 2; or
(2) an alkylene group as defined above that is interrupted by 1-20 atoms independently chosen from oxygen, sulfur and NRₐ-, where Rₐ is chosen from hydrogen, optionally substituted alkyl, cycloalkyl, cycloalkenyl, aryl, heteroaryl and heterocycyl, or groups selected from carbonyl, carboxyester, carboxyamide and sulfonyl; or
(3) an alkylene group as defined above that has both 1, 2, 3, 4 or 5 substituents as defined above and is also interrupted by 1-20 atoms as defined above. Examples of substituted alkylenes are chloromethylene (-CH(Cl)-), aminoethylene (-CH(NH₂)CH₂-), methylaminoethylene (-CH(NHMe)CH₂-), 2-carboxypropylene isomers (-CH₂CH(CO₂H)CHO₂-), ethoxyethyl (-CH₂CH₂O-CH₂CH₂-), ethylmethylaminoethyl (-CH₂CH₂N(CH₃)CH₂CH₂-), 1-ethoxy-2-(2-ethoxy-ethoxy)ethane (-CH₂CH₂O-CH₂CH₂OCH₂CH₂OCH₂CH₂-).

The term "aralkyl" refers to an aryl group covalently linked to an alkylene group, where aryl and alkylene are defined herein. "Optionally substituted aralkyl" refers to an optionally substituted aryl group covalently linked to an optionally substituted alkylene group. Such aralkyl groups are exemplified by benzyl, phenylethyl, 3-(4-methoxyphenyl)propyl.

The term "alkoxy" refers to the group R-O-, where R is optionally substituted alkyl or optionally substituted cycloalkyl, or R is the group -Y-Z, in which Y is optionally substituted alkylene and Z is optionally substituted alkenyl, optionally substituted alkynyl; or optionally substituted cycloalkenyl, where alkyl, alkenyl, alkynyl, cycloalkyl and cycloalkenyl are as defined herein. Preferred alkoxy groups are optionally substituted alkyl-O- and include, by way of example, methoxy, ethoxy, n-propoxy, iso-propoxy, n-butoxy, tert-butoxy, sec-butoxy, n-pentoxy, n-hexoxy, 1,2-dimethylbutoxy, trifluoromethoxy.

The term "alkylthio" refers to the group R-S-, where R is as defined for alkoxy.

The term "alkenyl" refers to a monoradical of a branched or unbranched unsaturated hydrocarbon group preferably having from 2 to 20 carbon atoms, more preferably 2 to 10 carbon atoms and even more preferably 2 to 6 carbon atoms and having 1-6, preferably 1, double bond (vinyl). Preferred alkenyl groups include ethenyl or vinyl (-CH=CH₂), 1-propylene or allyl (-CH₂CH=CH₂), isopropylene (-C(CH₃)=CH₂), bicyclo[2.2.1]heptene. In the event that alkenyl is attached to nitrogen, the double bond cannot be alpha to the nitrogen.

The term "lower alkenyl" refers to alkenyl as defined above having from 2 to 6 carbon atoms.

The term "substituted alkenyl" refers to an alkenyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, -SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "alkynyl" refers to a monoradical of an unsaturated hydrocarbon, preferably having from 2 to 20 carbon atoms, more preferably 2 to 10 carbon atoms and even more preferably 2 to 6 carbon atoms and having at least 1 and preferably from 1-6 sites of acetylene (triple bond) unsaturation. Preferred alkynyl groups include ethynyl, (-C≡CH), propargyl (or prop-1-yn-3-yl, -CH₂C≡CH). In the event that alkynyl is attached to nitrogen, the triple bond cannot be alpha to the nitrogen.

The term "substituted alkynyl" refers to an alkynyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, , aminocarbonylamino, heteroaryloxy, heterocyclyl, heterocyclooxy, hydroxyamino, alkoxyamino, nitro, -SO-alkyl, -SO-aryl, -SO-heteraryl, -SO₂-alkyl, -SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 our 2.

The term "aminocarbonyl" refers to the group -C(O)NRR where each R is independently hydrogen, alkyl, aryl, heteroaryl, heterocyclyl or where both R groups are joined to form a heterocyclic group (e.g., morpholino). Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "acylamino" refers to the group NRC(O)R where each R is independently hydrogen, alkyl, aryl, heteroaryl, or heterocyclyl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "acyloxy" refers to the groups -O(O)C-alkyl, -O(O)C-cycloalkyl, -O(O)C-aryl, -O(O)C-heteroaryl, and -O(O)C-heterocyclyl. Unless othervvise constrained by the definition, all substituents may be optionally further substituted by alkyl, carboxy, carboxyalkyl, aminocarbonyl, hydroxy, alkoxy, halogen, CF₃, amino, substituted amino, cyano, or -S(O)ₙR, where R is alkyl, aryl, or heteroaryl and n is 0, 1 or 2.

The term "aryl" refers to an aromatic carbocyclic group of 6 to 20 carbon atoms having a single ring (e.g., phenyl) or multiple rings (e.g., biphenyl), or multiple condensed (fused) rings (e.g., naphthyl or anthryl). Preferred aryls include phenyl and naphthyl.

The term "arylene" refers to a diradical of an aryl group as defined above. This term is exemplified by groups such as 1,4-phenylene, 1,3-phenylene, 1,2-phenylene and 1,4'-biphenylene.

Unless otherwise constrained by the definition for the aryl or arylene substituent, such aryl or arylene groups can optionally be substituted with from 1 to 5 substituents, preferably 1 to 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, -SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0,1 or 2.

The term "aryloxy" refers to the group aryl-O- wherein the aryl group is as defined above, and includes optionally substituted aryl groups as also defined above. The term "arylthio" refers to the group R-S-, where R is as defined for aryl.

The term "amino" refers to the group -NH₂.

The term "substituted amino" refers to the group -NRR where each R is independently selected from the group consisting of hydrogen, alkyl, cycloalkyl, carboxyalkyl (for example, benzyloxycarbonyl), aryl, heteroaryl and heterocyclyl provided that both R groups are not hydrogen, or a group -Y-Z, in which Y is optionally substituted alkylene and Z is alkenyl, cycloalkenyl, or alkynyl, Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "carboxyalkyl" refers to the groups -C(O)O-alkyl or -C(O)O-cycloalkyl, where alkyl and cycloalkyl, are as defined herein, and may be optionally further substituted by alkyl, alkenyl, alkynyl, alkoxy, halogen, CF₃, amino, substituted amino, cyano, or -S(O)ₙR, in which R is alkyl, aryl, or heteroaryl and n is 0, 1 or 2.

The term "cycloalkyl" refers to carbocyclic groups of from 3 to 20 carbon atoms having a single cyclic ring or multiple condensed rings. Such cycloalkyl groups include, by way of example, single ring structures such as cyclopropyl, cyclobutyl, cyclopentyl, cyclooctyl, or multiple ring structures such as adamantanyl, bicyclo[2.2.1]heptane, 1,3,3-trimethylbicyclo[2.2.1]hept-2-yl, (2,3,3-trimethylbicyclo[2.2.1]hept-2-yl), or carbocyclic groups to which is fused an aryl group, for example indane.

The term "substituted cycloalkyl" refers to cycloalkyl groups having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, -SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "halogen" or "halo" refers to fluoro, bromo, chloro, and iodo.

The term "acyl" denotes the group -C(O)R, in which R is hydrogen, optionally substituted alkyl, optionally substituted cycloalkyl, optionally substituted heterocyclyl, optionally substituted aryl, and optionally substituted heteroaryl..

The term "heteroaryl" refers to a radical derived from an aromatic cyclic group (i.e., fully unsaturated) having 1, 2, 3, 4, 5, 6, 7, 8, 9, 10, 11, 12, 13, 14, or 15 carbon atoms and 1, 2, 3 or 4 heteroatoms selected from oxygen, nitrogen and sulfur within at least one ring. Such heteroaryl groups can have a single ring (e.g., pyridyl or furyl) or multiple condensed rings (e.g., indolizinyl, benzothiazolyl, or benzothienyl). Examples of heteroaryls include, but are not limited to, [1,2,4]oxadiazole, [1,3,4]oxadiazole, [1,2,4]thiadiazole, [1,3,4]thiadiazole, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, as well as N-oxide and N-alkoxy derivatives of nitrogen containing heteroaryl compounds, for example pyridine-N-oxide derivatives.

The term "heteroarylene" refers to a diradical of a heteroaryl group as defined above. This term is exemplified by groups such as 2,5-imidazolene, 3,5-[1,2,4]oxadiazolene, 2,4-oxazolene, 1,4-pyrazolene. For example, 1,4-pyrazolene is: 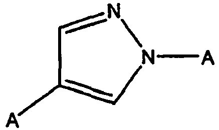 where A represents the point of attachment.

Unless otherwise constrained by the definition for the heteroaryl or heteroarylene substituent, such heteroaryl or heterarylene groups can be optionally substituted with 1 to 5 substituents, preferably 1 to 3 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, -SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "heteroaralkyl" refers to a heteroaryl group covalently linked to an alkylene group, where heteroaryl and alkylene are defined herein. "Optionally substituted heteroaralkyl" refers to an optionally substituted heteroaryl group covalently linked to an optionally substituted alkylene group. Such heteroaralkyl groups are exemplified by 3-pyridylmethyl, quinolin-8-ylethyl, 4-methoxythiazol-2-ylpropyl.

The term "heteroaryloxy" refers to the group heteroaryl-O-.

The term "heterocyclyl" refers to a monoradical saturated or partially unsaturated group having a single ring or multiple condensed rings, having from 1 to 40 carbon atoms and from 1 to 10 hetero atoms, preferably 1, 2, 3 or 4 heteroatoms, selected from nitrogen, sulfur, phosphorus, and/or oxygen within the ring. Heterocyclic groups can have a single ring or multiple condensed rings, and include tetrahydrofuranyl, morpholino, piperidinyl, piperazino, dihydropyridino.

Unless otherwise constrained by the definition for the heterocyclic substituent, such heterocyclic groups can be optionally substituted with 1, 2, 3, 4 or 5, and preferably 1, 2 or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, -(SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR, where R is alkyl, aryl, or heteroaryl and n is 0, 1 or 2.

The term "thiol" refers to the group -SH.

The term "substituted alkylthio" refers to the group -S-substituted alkyl.

The term "heteroarylthiol" refers to the group -S-heteroaryl wherein the heteroaryl group is as defined above including optionally substituted heteroaryl groups as also defined above.

The term "sulfoxide" refers to the group -S(O)R, in which R is alkyl, aryl, or heteroaryl. "Substituted sulfoxide" refers to the group -S(O)R, in which R is substituted alkyl, substituted aryl, or substituted heteroaryl, as defined herein.

The term "sulfone" refers to the group -S(O)₂R, in which R is alkyl, aryl, or heteroaryl. "Substituted sulfone" refers to the group -S(O)₂R, in which R is substituted alkyl, substituted aryl, or substituted heteroaryl, as defined herein.

The term "keto" refers to the group -C(O)-.

The term "thiocarbonyl" refers to the group -C(S)-.

The term "carboxy" refers to the group -C(O)-OH.

"Optional" or "optionally" means that the subsequently described event or circumstance may or may not occur, and that the description includes instances where said event or circumstance occurs and instances in which it does not.

The term "compound of Formula I and Formula II" is intended to encompass the compounds used in the invention as disclosed, and the pharmaceutically acceptable salts, pharmaceutically acceptable esters, prodrugs, hydrates and polymorphs of such compounds. Additionally, the compounds used in the invention may possess one or more asymmetric centers, and can be produced as a racemic mixture or as individual enantiomers or diastereoisomers. The number of stereoisomers present in any given compound of Formula I depends upon the number of asymmetric centers present (there are 2ⁿ stereoisomers possible where n is the number of asymmetric centers). The individual stereoisomers may be obtained by resolving a racemic or non-racemic mixture of an intermediate at some appropriate stage of the synthesis, or by resolution of the compound of Formula I by conventional means. The individual stereoisomers (including individual enantiomers and diastereoisomers) as well as racemic and non-racemic mixtures of stereoisomers are encompassed within the scope of the present invention, all of which are intended to be depicted by the structures of this specification unless otherwise specifically indicated.

"Isomers" are different compounds that have the same molecular formula.

"Stereoisomers" are isomers that differ only in the way the atoms are arranged in space.

"Enantiomers" are a pair of stereoisomers that are non-superimposable mirror images of each other. A1:1 mixture of a pair of enantiomers is a "racemic" mixture. The term "(±)" is used to designate a racemic mixture where appropriate.

"Diastereoisomers" are stereoisomers that have at least two asymmetric atoms, but which are not mirror-images of each other.

The absolute stereochemistry is specified according to the Cahn-Ingold-Prelog R-S system. When the compound is a pure enantiomer the stereochemistry at each chiral carbon may be specified by either R or S. Resolved compounds whose absolute configuration is unknown are designated (+) or (-) depending on the direction (dextro- or laevorotary) which they rotate the plane of polarized light at the wavelength of the sodium D line.

"Topical administration" shall be defined as the delivery of the therapeutic agent to the surface of the wound and adjacent epithelium.

"Parenteral administration" is the systemic delivery of the therapeutic agent via injection to the patient.

The term "therapeutically effective amount" refers to that amount of a compound of Formula I that is sufficient to effect treatment, as defined below, when administered to a mammal in need of such treatment. The therapeutically effective amount will vary depending upon the specific activity of the therapeutic agent being used, the wound type (mechanical or thermal, full or partial thickness, etc.), the size of the wound, the wound's depth (if full thickness), the absence or presence of infection, time elapsed since the injury's infliction, and the age, physical condition, existence of other disease states, and nutritional status of the patient. Additionally, other medication the patient may be receiving will effect the determination of the therapeutically effective amount of the therapeutic agent to administer.

The term "treatment" or "treating" means any treatment of a disease in a mammal, including:
(i) preventing the disease, that is, causing the clinical symptoms of the disease not to develop;
(ii) inhibiting the disease, that is, arresting the development of clinical symptoms; and/or
(iii) relieving the disease, that is, causing the regression of clinical symptoms.

In many cases, the compounds used in this invention are capable of forming acid and/or base salts by virtue of the presence of amino and/or carboxyl groups or groups similar thereto. The term "pharmaceutically acceptable salt" refers to salts that retain the biological effectiveness and properties of the compounds of Formula I, and which are not biologically or otherwise undesirable. Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group.

Specific examples of suitable amines include, by way of example only, isopropylamine, trimethyl amine, diethyl amine, tri(iso-propyl) amine, tri(n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid and salicylic acid.

As used herein, "pharmaceutically acceptable carrier" includes any and all solvents, dispersion media, coatings, antibacterial and antifungal agents, isotonic and absorption delaying agents. The use of such media and agents for pharmaceutically active substances is well known in the art Except insofar as any conventional media or agent is incompatible with the active ingredient, its use in the therapeutic compositions is contemplated. Supplementary active ingredients can also be incorporated into the compositions.

### Nomenclature

The naming and numbering of the compounds used in the invention is illustrated with a representative compound of Formula I in which R¹ is n-propyl, R² is n-propyl, R³ is hydrogen, X is phenylene, Y is -O-(CH₂), and Z is 5-(2-methoxyphenyl)-[1,2,4]-oxadiazol-3-yl, 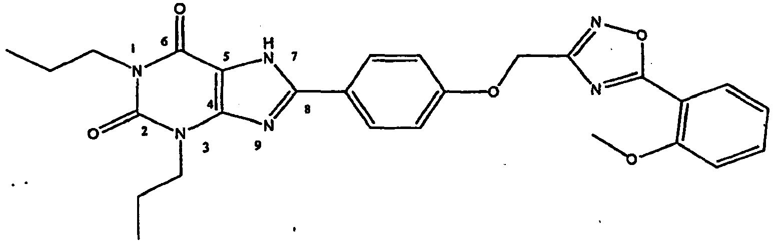 which is named:
8-{4-[5-(2-methoxyphenyl)-[1,2,4]-oxadiazol-3-ylmethoxy]phenyl}-1,3-dipropyl-1,3,7-trihydropurine-2,6-dione.

### The use of the Invention

The present invention relates to an A_{2B} adenosine receptor antagonist having the structure of Formula I or II in methods of augmenting wound healing. The wound being treated may be caused by mechanical, chemical or thermal means. The wound may be a contusion, incision or laceration. The wound may also be the result of a surgical incision. Alternatively, the wound may be associated with a disease or disorder, such as diabetes where the wound might take the form of a diabetic ulcer.

The A_{2B} adenosine receptor antagonist may be administered topically or systemically but will generally be topically administered to the wound site. This topical administration can be as a single dose or as repeated doses given at multiple designated intervals. It will readily be appreciated by those skilled in the art that the preferred dosage regimen will vary with the type and severity of the injury being treated.

When administered systemically, a therapeutically effective amount of the A_{2B} adenosine receptor antagonist is delivered by the parenteral route, i.e. by subcutaneous, intravenous, intramuscular, or intraperitoneal injection. Wound treatment by parenteral injection may involve either single, multiple, or continuous administration of the therapeutic agent, depending upon various factors, including the injury type, severity, and location.

### The Pharmaceutical Formulations

In a preferred embodiment, the A_{2B} adenosine receptor antagonist is incorporated into a pharmaceutical formulation containing a pharmaceutically acceptable carrier that is generally suited to topical drug administration and comprising any such material known in the art. Suitable carriers are well known to those of skill in the art and the selection of the carrier will depend upon the form of the intended pharmaceutical formulation, e.g., as an ointment, lotion, cream, foam, microemulsion, gel, oil, solution, spray, salve, and may be comprised of either naturally occurring or synthetic materials. It is understood that the selected carrier should not adversely affect the A_{2B} adenosine receptor antagonist or other components of the pharmaceutical formulation.

Suitable carriers for these types of formulations include, but are not limited to, vehicles including Shephard's^{™} Cream, Aquaphor^{™}, and Cetaphil^{™} lotion. Other preferred carriers include ointment bases, e.g., polyethylene glycol-1000 (PEG-1000), conventional creams such as HEB cream, gels, as well as petroleum jelly. Examples of suitable carriers for use herein include water, alcohols and other nontoxic organic solvents, glycerin, mineral oil, silicone, petroleum jelly, lanolin, fatty acids, vegetable oils, parabens, waxes. Particularly preferred formulations herein are colorless, odorless ointments, lotions, creams, microemulsions and gels.

Ointments are semisolid preparations that are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency. As with other carriers or vehicles, an ointment base should be inert, stable, nonirritating and nonsensitizing. As explained in Remington's Pharmaceutical Sciences, 20th Ed. (Easton, Pa.: Mack Publishing Company, 2000), ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin, and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. Preferred water-soluble ointment bases are prepared from polyethylene glycols (PEGs) of varying molecular weight; again, reference may be had to Remington's, supra, for further information.

Lotions are preparations to be applied to the skin surface without friction, and are typically liquid or semiliquid preparations in which solid particles, including the active agent, are present in a water or alcohol base. Lotions are usually suspensions of solids, and preferably, for the present purpose, comprise a liquid oily emulsion of the oil-in-water type. Lotions are preferred formulations herein for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose. A particularly preferred lotion formulation for use in conjunction with the present invention contains propylene glycol mixed with a hydrophilic petrolatum such as that which may be obtained under the trademark Aquaphor^{™} from Beiersdorf, Inc. (Norwalk, Conn.).

Creams containing the active agent are, as known in the art, viscous liquid or semisolid emulsions, either oil-in-water or water-in-oil. Cream bases are water-washable, and contain an oil phase, an emulsifier, and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant The emulsifier in a cream formulation, as explained in Remington's, supra, is generally a nonionic, anionic, cationic, or amphoteric surfactant

Microemulsions are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, a surfactant (emulsifier), a co-surfactant (co-emulsifier), an oil phase, and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of polyglycerol derivatives, glycerol derivatives, and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol,
while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

Gel formulations are semisolid systems consisting of either small inorganic particle suspensions (two-phase systems) or large organic molecules distributed substantially uniformly throughout a carrier liquid (single phase gels). Single phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulose, polyoxyethylene-polyoxypropylene, hydroxyethylcellulose and gelatin. Although gels commonly employ aqueous carrier liquid, alcohols and oils can be used as the carrier liquid as well.

Various additives, known to those skilled in the art, may be included in the topical formulations of the invention. Examples of additives include, but are not limited to, solubilizers, skin permeation enhancers, opacifiers, preservatives (e.g., antioxidants), gelling agents, buffering agents, surfactants (particularly nonionic and amphoteric surfactants), emulsifiers, emollients, thickening agents, stabilizers, humectants, colorants and fragrance. Inclusion of solubilizers and/or skin permeation enhancers is particularly preferred, along with emulsifiers, emollients, and preservatives.

Examples of solubilizers include, but are not limited to, the following: hydrophilic ethers such as diethylene glycol monoethyl ether (ethoxydiglycol, available commercially as Transcutol^{™}) and diethylene glycol monoethyl ether oleate (available commercially as Softcutol^{™}); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol^{™}); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate and polypropylene glycol-2 (PPG-2) myristyl ether propionate.

Other active agents may also be included in the formulation, e.g., antiinflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (e.g., octyl methoxycinnamate), dibenzoyl methanes (e.g., butyl methoxydibenzoyl methane), p-aminobenzoic acid (PABA) and derivatives thereof, and salicylates (e.g., octyl salicylate).

In the preferred topical formulations of the invention, the active agent is present in an amount in the range of 0.25 wt. % to 75 wt. % of the formulation, preferably in the range of 0.25 wt. % to 30 wt. % of the formulation, more preferably in the range of 0.5 wt. % to 15 wt. % of the formulation, and most preferably in the range of 1.0 wt. % to 10 wt. % of the formulation.

Also, the pharmaceutical formulation may be sterilized or mixed with auxiliary agents, e.g., preservatives, stabilizers, wetting agents, buffers, or salts for influencing osmotic pressure. Sterile injectable solutions are prepared by incorporating the compound of Formula I or Formula II in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

### The A_{2B} Adenosine Receptor Antagonists

Any A_{2B} adenosine receptors antagonist having the structure of Formula I or II may be used according to the invention. Numerous methods for determining if a specific compound has such activity are known in the art. For example, a review article by Feoktistov and Baggioni, (Pharmacological Reviews 49, 381-402 (1997)) reports the binding affinity of eight adenosine receptor agonists and eight antagonists for all four subtypes of adenosine receptors. References cited therein provide detailed descriptions of the procedures used. (Robeva A. S., Woodward R. L., Jin X. and Gao Z., Linden J. Drug Dev. Res 39:243-252 (1996); Jacobson K. A. and Suzuki F. Drug Dev. Res. 39, 289-300, (1996); Feoktistov, L and Baggioni, L Molecular Pharmacology 43, 909-914 (1993)). Effective methods for determining the binding affinity of a compound for a receptor use a radiolabelled agonist or antagonist and correlation of the binding of that compound to a membrane fraction known to contain that receptor; for example, to determine whether a compound is an A_{2B} antagonist, the membrane fraction would contain the A_{2B} adenosine receptor. Another particularly effective procedure for determining whether a compound is an A_{2B} antagonist is reported in U.S. Patent No. 5,854,081.

Compounds selective for the A_{2B} receptor subtype are therefore preferred for the present invention. Compounds that antagonize other receptors in addition to the A_{2B} receptor are also suitable for use in the present invention.

### Synthetic Reaction Parameters

The terms "solvent", "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform, methylene chloride (or dichloromethane), diethyl ether, methanol, pyridine]. Unless specified to the contrary, the solvents used in the reactions described herein are inert organic solvents, and the reactions are carried out under an inert gas, preferably nitrogen.

The term "q.s." means adding a quantity sufficient to achieve a stated function, e.g., to bring a solution to the desired volume (i.e., 100%).

### Synthesis of the Compounds of Formula I and II

One preferred method of preparing compounds of Formula I or II where R³ is hydrogen is shown in Reaction Scheme I.

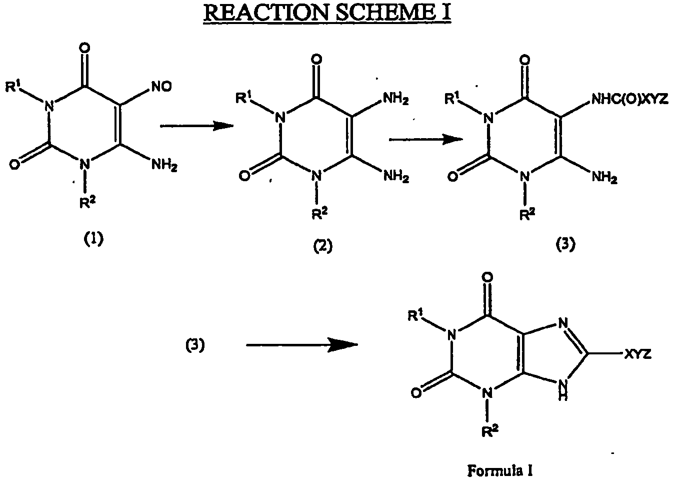

### Step 1 - Preparation of Formula (2)

The compound of formula (2) is made from the compound of formula (1) by a reduction step. Conventional reducing techniques may be used, for example using sodium dithionite in aqueous ammonia solution; preferably, reduction is carried out with hydrogen and a metal catalyst. The reaction is carried out at in an inert solvent, for example methanol, in the presence of a catalyst, for example 10% palladium on carbon catalyst, under an atmosphere of hydrogen, preferably under pressure, for example at 30 psi, for 2 hours. When the reaction is substantially complete, the product of formula (2) is isolated by conventional means to provide a compound of formula (2).

### Step 2 - Preparation of Formula (3)

The compound of formula (2) is then reacted with a carboxylic acid of the formula Z-Y-X-CO₂H in the presence of a carbodiimide, for example 1-(3-dimethylaminopropyl)-3-ethylcarbodiimide hydrochloride. The reaction is conducted in a protic solvent, for example methanol, ethanol, propanol, preferably methanol, at a temperature of 20-30°C, preferably room temperature, for 12-48 hours, preferably 16 hours. When the reaction is substantially complete, the product of formula (3) is isolated conventionally, for example by removal of the solvent under reduced pressure, and washing the product. Alternatively, the next step can be carried out without any further purification.

### Alternative Preparation of a Compound of Formula (3)

Alternatively, the carboxylic acid of the formula Z-Y-X-CO₂H is first converted to an acid halide of the formula Z-Y-X-C(O)L, where L is chloro or bromo, by reacting with a halogenating agent, for example thionyl chloride or thionyl bromide, preferably thiony chloride. Alternatively, oxalyl chloride, phosphorus pentachloride or phosphorus oxychloride may be used. The reaction is preferably conducted in the absence of a solvent, using excess halogenating agent, for example at a temperature of 60-80°C, preferably 70°C, for 1-8 hours, preferably 4 hours. When the reaction is substantially complete, the product of formula Z-Y-X-C(O)L is isolated conventionally, for example by removal of the excess halogenating agent under reduced pressure.

The product is then reacted with a compound of formula (2) in an inert solvent, for example acetonitrile, in the presence of a tertiary base, for example triethylamine. The reaction is conducted at an initial temperature of 0°C, and then allowed to warm to 20-30°C, preferably room temperature, for 12-48 hours, preferably 16 hours. When the reaction is substantially complete, the product of formula (3) is isolated conventionally, for example by diluting the reaction mixture with water, filtering off the product, and washing the product with water followed by ether.

### Step 3 - Preparation of Formula I

The compound of formula (3) is then converted into a compound of Formula I by a cyclization reaction. The reaction is conducted in a protic solvent, for example methanol, ethanol and propanol, preferably methanol, in the presence of a base, for example potassium hydroxide, sodium hydroxide, sodium methoxide, sodium ethoxide, potassium t-butoxide, preferably aqueous sodium hydroxide, at a temperature of 50-80°C, preferably 80°C, for 1-8 hours, preferably 3 hours. When the reaction is substantially complete, the product of Formula I is isolated conventionally, for example by removal of the solvent under reduced pressure, acidifying the residue with an aqueous acid, filtering off the product, then washing and drying the product.

The compound of formula (1) may be prepared by various methods. One preferred method is shown in Reaction Scheme II.

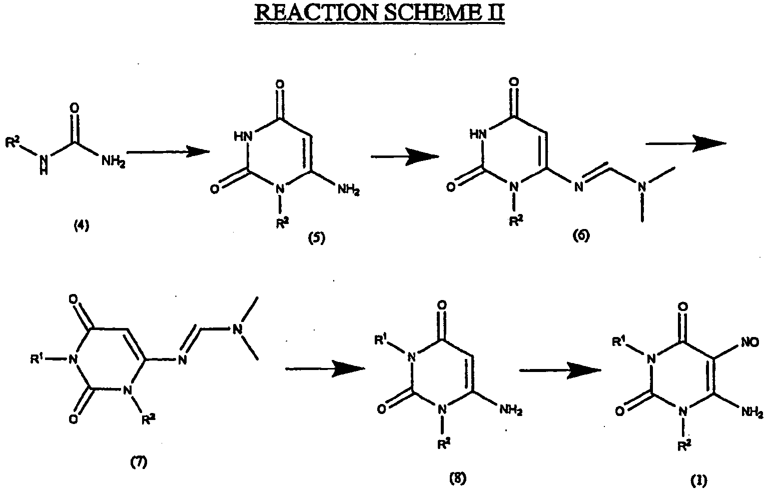

### Step 1 - Preparation of Formula (5)

The compound of formula (4) is either commercially available or prepared by means well known in the art. It is reacted with ethyl cyanoacetate in a protic solvent, for example ethanol, in the presence of a strong base, for example sodium ethoxide. The reaction is carried out at reflux temperature, for 4 to 24 hours. When the reaction is substantially complete, the compound of formula (5) thus produced is isolated conventionally.

### Steps 2 and 3 - Preparation of Formula (7)

The compound of formula (5) is reacted with the dimethylacetal of N,N-dimethylformamide in a polar solvent, for example N,N-dimethylformamide. The reaction is carried out at 40°C, for 1 hour. When the reaction is substantially complete, the compound of formula (6) thus produced is reacted with a compound of formula R¹Hal, where Hal is chloro, bromo, or iodo, in the presence of a base, for example potassium carbonate. The reaction is carried out at 80°C, for 4-24 hour. When the reaction is substantially complete, the product of formula (7) is isolated conventionally, for example by evaporation of the solvents under reduced pressure, and the residue is used in the next reaction with no further purification.

### Step 4 - Preparation of Formula (8)

The compound of formula (7) is reacted with aqueous ammonia in a polar solvent, for example suspended in methanol. The reaction is carried out at room temperature, for 1-3 days. When the reaction is substantially complete, the product of formula (8) is isolated conventionally, for example by chromatography over a silica gel column, eluting, for example, with a mixture of dichloromethane/methanol.

### Step 5 - Preparation of Formula (1)

The compound of formula (8) is then mixed with sodium nitrite in an aqueous acidic solvent, preferably acetic acid and water, for example 50% acetic acid/water. The reaction is carried out at a temperature of 50-90°C, preferably 70°C, for 1 hour. When the reaction is substantially complete, the product of formula (1) is isolated by conventional means.

Alternatively, the reaction may be conducted in an aqueous solvent, for example dimethylformamide and water, and reacted with a strong acid, for example hydrochloric acid.

A compound of formula (8) can be prepared from a compound of formula (10) using a similar method, as shown in Reaction Scheme IIA.

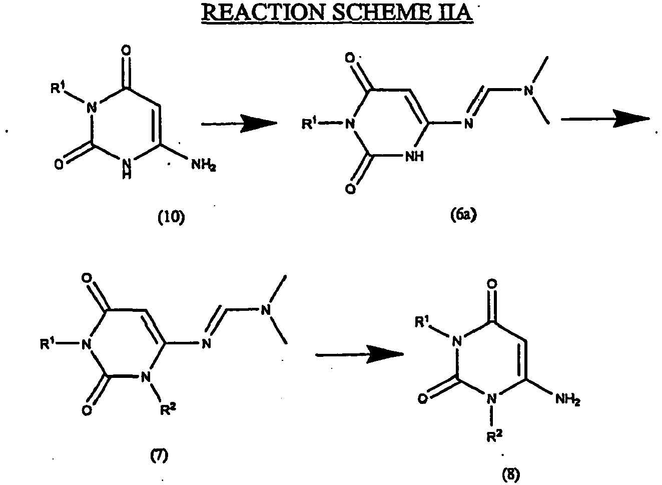

### Steps 2 and 3 - Preparation of Formula (7)

The compound of formula (10) is reacted with the dimethylacetal ofN,N-dimethylformamide in a polar solvent, for example N,N-dimethylformamide. The reaction is carried out at 40°C, for 1 hour. When the reaction is substantially complete, the compound of formula (6a) thus produced is reacted with a compound of formula R²Hal, where Hal is chloro, bromo, or iodo, in the presence of a base, for example potassium carbonate. The reaction is carried out at 80°C, for 4-24 hour. When the reaction is substantially complete, the product of formula (7) is isolated conventionally, for example by evaporation of the solvents under reduced pressure, and the residue is used in the next reaction with no further purification.

### Step 4 - Preparation of Formula (8)

The compound of formula (7) is reacted with aqueous ammonia in a polar solvent, for example suspended in methanol. The reaction is carried out at room temperature, for 1-3 days. When the reaction is substantially complete, the product of formula (8) is isolated conventionally, for example by chromatography over a silica gel column, eluting, for example, with a mixture of dichloromethane/methanol.

The compound of formula (3) may also be prepared by various methods. One preferred method is shown in Reaction Scheme III.

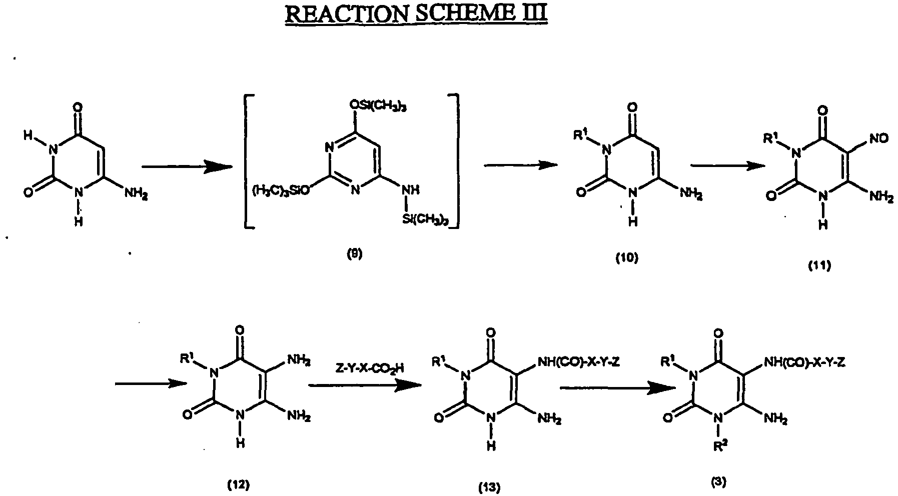

### Step 1 - Preparation of Formula (10)

The commercially available compound 6-aminouracil is first silylated, for example by reaction with excess hexamethyldisilazane as a solvent in the presence of a catalyst, for example ammonium sulfate. The reaction is carried out at about reflux temperature, for 1-10 hours. When the reaction is substantially complete, the silylated compound thus produced is isolated conventionally, and then reacted with a compound of formula R¹Hal, where Hal is chloro, bromo, or iodo, preferably in the absence of a solvent. The reaction is carried out at reflux, for 4-48 hours, preferably 12-16 hours. When the reaction is substantially complete, the product of formula (10) is isolated by conventional means.

### Step 2 - Preparation of Formula (11)

The compound of formula (10) is then dissolved in an aqueous acid, for example aqueous acetic acid, and reacted with sodium nitrite. The reaction is carried out at a temperature of 20-50°C, preferably 30°C, over 30 minutes. When the reaction is substantially complete, the product of formula (11) is isolated by conventional means, for example by filtration.

### Step 3 - Preparation of Formula (12)

The compound of formula (11) is then reduced to a diamino derivative. In general, the compound of formula (11) is dissolved in aqueous ammonia, and then a reducing agent, for example sodium hydrosulfite, added. The reaction is conducted at a temperature of 70°C. When the reaction is substantially complete, the product of formula (12) is isolated conventionally, for example by filtration of the cooled reaction mixture.

### Step 4 - Preparation of Formula (13)

The compound of formula (12) is then reacted with a carboxylic acid of the formula Z-Y-X-CO₂H in the presence of a carbodiimide, for example 1-(3-dimethylaminopropyl)-3-ethylcarbodiimide hydrochloride. The reaction is conducted at a temperature of 20-30°C, for 12-48 hours. When the reaction is substantially complete, the product of formula (13) is isolated conventionally, for example by filtration of the cooled reaction mixture.

Alternatively, the carboxylic acid of the formula Z-Y-X-CO₂H is converted to an acid halide of the formula Z-Y-X-C(O)L, where L is chloro or bromo, by reacting with a halogenating agent, for example thionyl chloride or thionyl bromide; alternatively, phosphorus pentachloride or phosphorus oxychloride may be used. The reaction is preferably conducted in the absence of a solvent, using excess halogenating agent, for example at a temperature of 60-80°C, preferably 70°C, for 1-8 hours, preferably 4 hours. When the reaction is substantially complete, the product of formula Z-Y-X-C(O)L is isolated conventionally, for example by removal of the excess halogenating agent under reduced pressure.

The product of the formula Z-Y-X-C(O)L is then reacted with a compound of formula (12) in an inert solvent, for example acetonitrile, in the presence of a tertiary base, for example triethylamine. The reaction is conducted at an initial temperature of 0°C, and then allowed to warm to 20-30°C, preferably room temperature, for 12-48 hours, preferably 16 hours. When the reaction is substantially complete, the product of formula (13) is isolated conventionally, for example by diluting the reaction mixture with water, filtering off the product, and washing the product with water followed by ether.

### Step 5 - Preparation of Formula (3)

The compound of formula (13) is reacted with a compound of formula R²Hal, where Hal is chloro, bromo, or iodo, in the presence of a base, for example potassium carbonate. The reaction is carried out at room temperature, for 4-24 hour, preferably 16 hours. When the reaction is substantially complete, the product of formula (3) is isolated conventionally, for example by evaporation of the solvents under reduced pressure, and the residue may be purified conventionally, or may be used in the next reaction with no further purification.

Another method of preparing a compound of formula (3) is shown in Reaction Scheme IV.

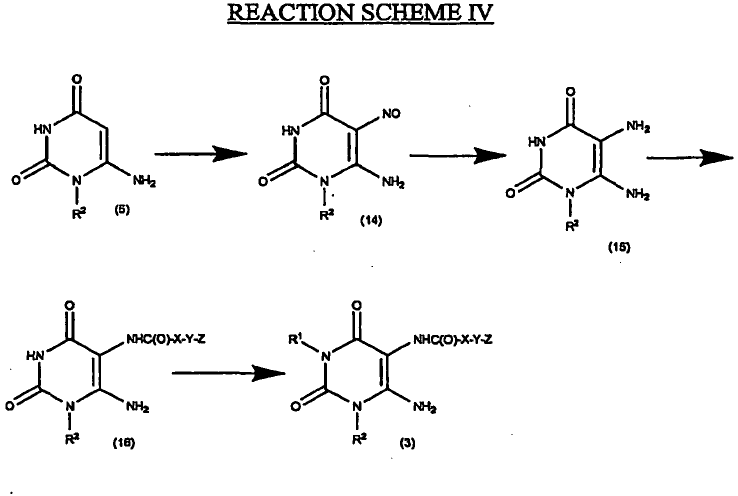

### Step 1 - Preparation of Formula (14)

The compound of formula (5) is mixed with sodium nitrite in an aqueous acidic solvent, preferably acetic acid and water, for example 50% acetic acid/water. The reaction is carried out at a temperature of 50-90°C, preferably 70°C, for 1 hour. When the reaction is substantially complete, the product of formula (14) is isolated by conventional means.

Alternatively, the reaction may be conducted in an aqueous solvent, for example dimethylformamide and water, and reacted with a strong acid, for example hydrochloric acid.

### Step 2 - Preparation of Formula (15)

The compound of formula (14) is then reduced to a diamino derivative. In general, the compound of formula (14) is dissolved in aqueous ammonia, and then a reducing agent, for example sodium hydrosulfite, added. The reaction is conducted at a temperature of 70°C. When the reaction is substantially complete, the product of formula (15) is isolated conventionally, for example by filtration of the cooled reaction mixture.

### Step 3 - Preparation of Formula (16)

The compound of formula (15) is then reacted with a carboxylic acid of the formula Z-Y-X-CO₂H in the presence of a carbodiimide, for example 1-(3-dimethylaminopropyl)-3-ethylcarbodiimide hydrochloride. The reaction is conducted at a temperature of 20-30°C, for 12-48 hours, in an inert solvent, for example methanol. When the reaction is substantially complete, the product of formula (16) is isolated conventionally, for example by filtration of the cooled reaction mixture.

Alternatively, the carboxylic acid of the formula Z-Y-X-CO₂H is converted to an acid halide of the formula Z-Y-X-C(O)L, where L is chloro or bromo, by reacting with a halogenating agent, for example thionyl chloride or thionyl bromide; alternatively, phosphorus pentachloride or phosphorus oxychloride may be used. The reaction is preferably conducted in the absence of a solvent, using excess halogenating agent, for example at a temperature of 60-80°C, preferably 70°C, for 1-8 hours, preferably 4 hours. When the reaction is substantially complete, the product of formula Z-Y-X-C(O)L is isolated conventionally, for example by removal of the excess halogenating agent under reduced pressure.

The product of the formula Z-Y-X-C(O)L is then reacted with a compound of formula (15) in an inert solvent, for example acetonitrile, in the presence of a tertiary base, for example triethylamine. The reaction is conducted at an initial temperature of 0°C, and then allowed to warm to 20-30°C, preferably room temperature, for 12-48 hours, preferably 16 hours. When the reaction is substantially complete, the product of formula (16) is isolated conventionally, for example by diluting the reaction mixture with water, filtering off the product, and washing the product with water followed by ether.

### Step 4 - Preparation of Formula (3)

The compound of formula (16) is reacted with a compound of formula R¹Hal, where Hal is chloro, bromo, or iodo, in the presence of a base, for example potassium carbonate. The reaction is carried out at 80°C, for 4-24 hour, preferably 16 hours. When the reaction is substantially complete, the product of formula (3) is isolated conventionally, for example by evaporation of the solvents under reduced pressure, and the residue may be purified conventionally, or may be used in the next reaction with no further purification.

An example of a synthesis of a compound of Z-Y-X-CO₂H in which X is pyrazol-1,4-yl, Y is methylene, and Z is 3-trifluoromethylphenyl, is shown in Reaction Scheme V.

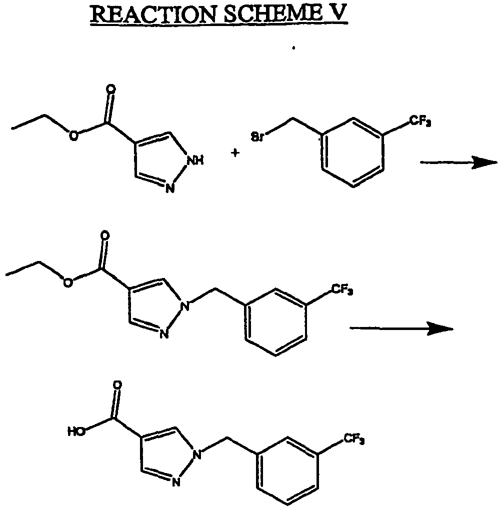

Ethyl pyrazole-4-carboxylate is reacted with 1-(bromomethyl)-3-(trifluoromethyl)benzene in acetone in the presence of potassium carbonate. The product, ethyl 1-{[3-(trifluoromethyl)phenyl]methyl}pyrazole-4-carboxylate, is then hydrolyzed with potassium hydroxide in methanol, to provide 1-{[3-(trifluoromethyl)phenyl]methyl}pyrazole-4-carboxylic acid.

### Utility, Testing and Administration

### General Utility

The compounds having the structure of Formula I or II and pharmaceutical compositions of the invention are effective in the augmentation of wound healing.

### Testing

Activity testing is conducted as described in those patents and patent applications referenced above, and in the Examples below, and by methods apparent to one skilled in the art.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1

### Preparation of a Compound of Formula (5)

### A. Preparation of a Compound of Formula (5) in which R² is Ethyl

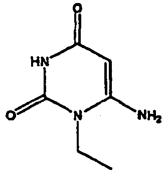

A solution of sodium ethoxide was prepared from sodium (4.8g, 226 mmol) and dry ethanol (150 ml). To this solution was added amino-N-ethylamide (10g, 113 mmol) and ethyl cyanoacetate (12.8 g, 113 mmol). This reaction mixture was stirred at reflux for 6 hours, cooled, and solvent removed from the reaction mixture under reduced pressure. The residue was dissolved in water (50 ml), and the pH adjusted to 7 with hydrochloric acid. The mixture was allowed to stand overnight at 0°C, and the precipitate filtered off, washed with water and air-dried, to provide 6-amino-1-ethyl-1,3-dihydropyrimidine-2,4-dione, a compound of formula (5).
¹H-NMR (DMSO-d6) δ 10.29 (s, 1H), 6.79 (s, 2H), 4.51 (s, 1H), 3.74-3.79 (m, 2H), 1.07 (t, 3H, J = 7.03 Hz); MS m/z 155.98 (M⁺), 177.99 (M⁺ +Na)

### B. Preparation of a Compound of Formula (5) in which R² is Methyl

Similarly, following the procedure of Example 1A, but replacing amino-N-ethylamide with amino-N-methylamide, 6-amino-1-methyl-1,3-dihydropyrimidine-2,4-dione was prepared.

### C. Preparation of a Compound of Formula (5) varying R²

Similarly, following the procedure of Example 1A, but replacing amino-N-ethylamide with other compounds of formula (4), other compounds of formula (5) are prepared.

### EXAMPLE 2

### Preparation of a Compound of Formula (6)

### A. Preparation of a Compound of Formula (6) in which R² is Ethyl

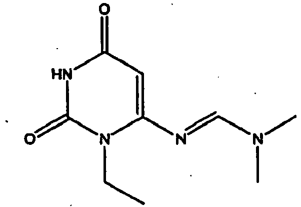

A suspension of 6-amino-1-ethyl-1,3-dihydropyrimidine-2,4-dione (0.77 g, 5 mmol) in anhydrous N,N-dimethylacetamide (25 ml) and N,N-dimethylformamide dimethylacetal (2.7 ml, 20 mmol) and was warmed at 40°C for 90 minutes. Solvent was then removed under reduced pressure, and the residue triturated with ethanol, filtered, and washed with ethanol, to provide 6-[2-(dimethylamino)-1-azavinyl]-1-ethyl-1,3-dihydropyrimidine-2,4-dione, a compound of formula (6).
¹H-NMR (DMSO-d6) 8 10.62 (s, 1H), 8.08 (s, 1H), 4.99 (s, 1H), 3.88-3.95 (m, 2H), 3.13 (s, 3H), 2.99 (s, 3H), 1.07 (t, 3H, J = 7.03 Hz); MS m/z 210.86 (M⁺), 232.87 (M⁺ +Na)

### B. Preparation of a Compound of Formula (6) in which R² is Methyl

Similarly, following the procedure of Example 2A, but replacing 6-amino-1-ethyl-1,3-dihydropyrimidine-2,4-dione with 6-amino-1-methyl-1,3-dihydropyrimidine-2,4-dione, 6-[2-(dimethylamino)-1-azavinyl]-1-methyl-1,3-dihydropyrimidine-2,4-dione was prepared.

### C. Preparation of a Compound of Formula (6) varying R²

Similarly, following the procedure of Example 2A, but replacing 6-amino-1-ethyl-1,3-dihydropyrimidine-2,4-dione with other compounds of formula (5), other compounds of formula (6) are prepared.

### EXAMPLE 3

### Preparation of a Compound of Formula (7)

### A. Preparation of a Compound of Formula (2) in which R¹ is n-Propyl and R² is Ethyl

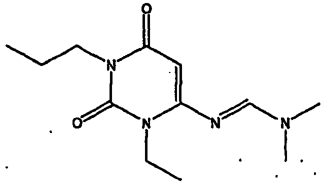

A mixture of a solution of 6-[2-(dimethylamino)-1-azavinyl]-1-ethyl-1,3-dihydropyrimidine-2,4-dione (1.5 g, 7.1 mmol) in dimethylformamide (25 ml), potassium carbonate (1.5 g, 11 mmol) and n-propyl iodide (1.54 g, 11 mmol) was stirred at 80°C for 5 hours. The reaction mixture was cooled to room temperature, filtered, the solvents were evaporated and the product of formula (7), 6-[2-(dimethylamino)-1-azavinyl]-1-ethyl-3-propyl-1,3-dihydropyrimidine-2,4-dione, was used as such in the next reaction.

### B. Preparation of a Compound of Formula (7), varying R¹ and R²

Similarly, following the procedure of Example 3A, but replacing 6-[2-(dimethylamino)-1-azavinyl]-1-ethyl-1,3-dihydropyrimidine-2,4-dione with other compounds of formula (6), the following compounds of formula (7) were prepared:
6-[2-(dimethylamino)-1-azavinyl]-1-methyl-3-propyl-1,3-dihydropyrimidine-2,4-dione.
6-[2-(dimethylamino)-1-azavinyl]-1-methyl-3-cyclopropylmethyl-1,3-dihydropyrimidine-2,4-dione;
6-[2-(dimethylamino)-1-azavinyl]-1-ethyl-3-cyclopropylmethyl-1,3-dihydropyrimidine-2,4-dione;
6-[2-(dimethylamino)-1-azavinyl]-1-methyl-3-(2-methylpropyl)-1,3-dihydropyrimidine-2,4-dione; and
6-[2-(dimethylamino)-1-azavinyl]-1-ethyl-3-(2-methylpropyl)-1,3-dihydropyrimidine-2,4-dione.

### C. Preparation of a Compound of Formula (7), varying R¹ and R²

Similarly, following the procedure of Example 3A, but replacing 6-[2-(dimethylamino)-1-azavinyl]-1-ethyl-1,3-dihydropyrimidine-2,4-dione with other compounds of formula (6), other compounds of formula (7) are prepared.

### EXAMPLE 4

### Preparation of a Compound of Formula (8)

### A. Preparation of a Compound of Formula (8) in which R¹ is n-Propyl and R² is Ethyl

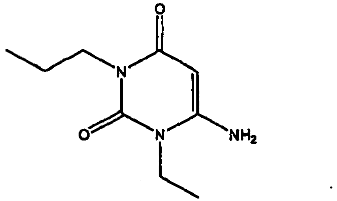

A solution of 6-[2-(dimethylamino)-1-azavinyl]-1-ethyl-3-propyl-1,3-dihydropyrimidine-2,4-dione (2.1 g) was dissolved in a mixture of methanol (10 ml) and 28% aqueous ammonia solution (20 ml), and stirred for 72 hours at room temperature. Solvent was then removed under reduced pressure, and the residue purified by chromatography on a silica gel column, eluting with a mixture of dichloromethane/methanol (15/1), to provide 6-amino-1-ethyl-3-propyl-1,3-dihydropyrimidine-2,4-dione, a compound of formula (8).
¹H-NMR (DMSO-d6) δ 6.80 (s, 2H), 4.64 (s, 1H), 3.79-3.84 (m, 2H), 3.63-3.67 (m, 2H), 1.41-1.51 (m, 2H), 1.09 (t, 3H, J = 7.03 Hz), 0.80 (t, 3H, J = 7.42 Hz); MS m/z 197.82 (M⁺)

### B. Preparation of a Compound of Formula (8), varying R¹ and R²

Similarly, following the procedure of Example 4A, but replacing 6-[2-(dimethylamino)-1-azavinyl]-1-ethyl-3-propyl-1,3-dihydropyrimidine-2,4-dione with other compounds of formula (7), the following compounds of formula (8) were prepared:
6-amino-1-methyl-3-propyl-1,3-dihydropyrimidine-2,4-dione;
6-amino-1-methyl-3-cyclopropylmethyl-1,3-dihydropyrimidine-2,4-dione;
6-amino-1-ethyl-3-cyclopropylmethyl-1,3-dihydropyrimidine-2,4-dione;
6-amino-1-methyl-3-(2-methylpropyl)-1,3-dihydropyrimidine-2,4-dione; and
6-amino-1-ethyl-3-(2-methylpropyl)-1,3-dihydropyrimidine-2,4-dione.

### C. Preparation of a Compound of Formula (7) varying R¹ and R²

Similarly, following the procedure of Example 4A, but replacing 6-[2-(dimethylamino)-1-azavinyl]-1-ethyl-3-propyl-1,3-dihydropyrimidine-2,4-dione with other compounds of formula (7), other compounds of formula (8) are prepared.

### EXAMPLE 5

### Preparation of a Compound of Formula (1)

### A. Preparation of a Compound of Formula (1) in which R¹ is n-Propyl and R² is Ethyl

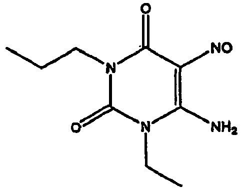

To a solution of 6-amino-1-ethyl-3-propyl-1,3-dihydropyrimidine-2,4-dione (1.4 g, 7.1 mmol) in a mixture of 50% acetic acid/water (35 ml) was added sodium nitrite (2 g, 28.4 mmol) in portions over a period of 10 minutes. The mixture was stirred at 70°C for 1 hour, then the reaction mixture concentrated to a low volume under reduced pressure. The solid was filtered off, and washed with water, to provide 6-amino-1-ethyl-5-nitroso-3-propyl-1,3-dihydropyrimidine-2,4-dione, a compound of formula (1).
MS *m*/*z* 227.05 (M⁺), 249.08 (M⁺ +Na)

### B. Preparation of a Compound of Formula (1), varying R¹ and R²

Similarly, following the procedure of Example 5A, but replacing 6-amino-1-ethyl-3-propyl-1,3-dihydropyrimidine-2,4-dione with other compounds of formula (8), the following compounds of formula (1) were prepared:
6-amino-1-methyl-5-nitroso-3-propyl-1,3-dihydropyrimidine-2,4-dione;
6-ammo-1-methyl-3-cyclopropylmethyl-5-nitroso-1,3-dihydropyrimidine-2,4-dione;
6-amino-1-ethyl-3-cyclopropylmethyl-5-nitroso-1,3-dihydropyrimidine-2,4-dione;
6-amino-1-methyl-3-(2-methylpropyl)-5-nitroso-1,3-dihydropyrimidine-2,4-dione; and
6-amino-1-ethyl-3-(2-methylpropyl)-5-nitroso-1,3-dihydropyrimidine-2,4-dione.

### C. Preparation of a Compound of Formula (1) varying R¹ and R²

Similarly, following the procedure of Example 5A, but replacing 6-amino-1-ethyl-3-propyl-1,3-dihydropyrimidine-2,4-dione with other compounds of formula (8), other compounds of formula (1) are prepared.

### EXAMPLE 6

### Preparation of a Compound of Formula (2)

### A. Preparation of a Compound of Formula (2) in which R¹ is n-Propyl and R² is Ethyl

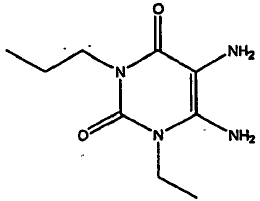

To a solution of 6-amino-1-ethyl-5-nitroso-3-propyl-1,3-dihydropyrimidine-2,4-dione (300 mg) in methanol (10 ml) was added 10% palladium on carbon catalyst (50 mg), and the mixture was hydrogenated under hydrogen at 30 psi for 2 hours. The mixture was filtered through celite, and solvent was removed from the filtrate under reduced pressure, to provide 5,6-diamino-1-ethyl-3-propyl-1,3-dihydropyrimidine-2,4-dione, a compound of formula (2).
MS *m*/*z* 213.03 (M⁺), 235.06 (M⁺ +Na)

### B. Preparation of a Compound of Formula (2), varving R¹ and R²

Similarly, following the procedure of Example 6A, but replacing 6-amino-1-ethyl-5-nitroso-3-propyl-1,3-dihydropyrimidine-2,4-dione with other compounds of formula (1), the following compounds of formula (2) were prepared:
5,6-diamino-1-methyl-3-propyl-1,3-dihydropyrimidine-2,4-dione;
5,6-diamino-1-methyl-3-cyclopropylmethyl-1,3-dihydropyrimidine-2,4-dione;
5,6-diamino-1-ethyl-3-cyclopropylmethyl-1,3-dihydropyrimidine-2,4-dione;
5,6-amino-1-methyl-3-(2-methylpropyl)-1,3-dihydropyrimidine-2,4-dione; and
5,6-diammo-1-ethyl-3-(2-methylpropyl)-1,3-dihydropyrimidine-2,4-dione.

### C. Preparation of a Compound of Formula (2) varying R¹ and R²

Similarly, following the procedure of Example 6A, but replacing 6-amino-1-ethyl-5-nitroso-3-propyl-1,3-dihydropyrimidine-2,4-dione with other compounds of formula (1), other compounds of formula (2) are prepared.

### EXAMPLE 7

### Preparation of a Compound of Formula (3)

### A. Preparation of a Compound of Formula (3) in which R¹ is n-Propyl, R² is Ethyl, X is 1,4-Pyrazolyl, Y is Methylene, and Z is 3-Trifluoromethylphenyl

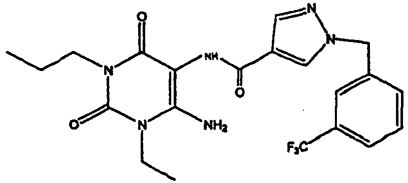

To a mixture of 5,6-diamino-1-ethyl-3-propyl-1,3-dihydropyrimidine-2,4-dione (100 mg, 0.47 mmol) and 1-{[3-(trifluoromethyl)phenyl]methyl}pyrazole-4-carboxylic acid (0.151 g, 0.56 mmol) in methanol (10 ml) was added 1-(3-dimethylaminopropyl)-3-ethylcarbodiimide hydrochloride (0.135 g, 0.7 mmol), and the reaction mixture was stirred overnight at room temperature. The solvent was removed under reduced pressure, and the residue purified using Bistag, eluting with 10% methanol/methylene chloride, to provide N-(6-amino-1-ethyl-2,4-dioxo-3-propyl(1,3-dihydropyrimidin-5-yl))(1-{[3-(trifluoromethyl)phenyl]methyl}-pyrazol-4-yl)carboxamide.
¹H-NMR (DMSO-d6) δ 8.59 (s, 1H), 8.02 (s, 1H), 7.59-7.71 (m, 4H), 6.71 (s, 2H), 5.51 (s, 2H), 3.91-3.96 (m, 2H), 3.70-3.75 (m, 2H), 1.47-1.55 (m, 2H),1.14 (t, 3H, J = 7.03 Hz), 0.85 (t, 3H, J = 7.42 Hz).

### B. Preparation of a Compound of Formula (3), varying R¹, R², X, Y, and Z

Similarly, following the procedure of Example 7A, but optionally replacing 5,6-diamino-1-ethyl-3-propyl-1,3-dihydropyrimidine-2,4-dione with other compounds of formula (2), and optionally replacing 1-{[3-(trifluoromethyl)phenyl]methyl}pyrazole-4-carboxylic acid with other compounds of formula Z-Y-X-CO₂H, the following compounds of formula (3) were prepared:
N-(6-amino-1-methyl-2,4-dioxo-3-propyl(1,3-dihydropyrimidin-5-yl))(1-{[3-(trifluoromethyl)phenyl]methyl}-pyrazol-4-yl)carboxamide;
N-(6-amino-1-methyl-2,4-dioxo-3-cyclopropylmethyl(1,3-dihydropyrimidin-5-yl))(1-{[3-(trifluoromethyl)phenyl]methyl} -pyrazol-4-yl)carboxamide;
N-(6-amino-1-ethyl-2,4-dioxo-3-cyclopropylmethyl(1,3-dihydropyrimidin-5-yl))(1-{[3-(trifluoromethyl)phenyl]methyl}-pyrazol-4-yl)carboxamide;
N-(6-amino-1-methyl-2,4-dioxo-3-ethyl(1,3-dihydropyrimidin-5-yl))(1-{[3-fluorophenyl]methyl}pyrazol-4-yl)carboxamide;
N-(6-amino-1-methyl-2,4-dioxo-3-cyclopropylmethyl(1,3-dihydropyrimidin-5-yl))(1-{[3-fluorophenyl]methyl}-pyrazol-4-yl)carboxamide;
N-(6-amino-1-ethyl-2,4-dioxo-3-cyclopropylmethyl(1,3-dihydropyrimidin-5-yl))(1-{[3-fluorophenyl]methyl}-pyrazol-4-yl)carboxamide;
N-[6-amino-3-(cyclopropylmethyl)-l-methyl-2,4-dioxo(1,3-dihydropyrimidin-5-yl)][1-benzylpyrazol-4-yl]carboxamide;
N-(6-amino-1-methyl-2,4-dioxo-3-cyclopropylmethyl(1,3-dihydropyrimidin-5-yl))(1-{[3-cyanophenyl]methyl}-pyrazol-4-yl)carboxamide;
[1-(2-(1H-1,2,3,4-tetrazol-5-yl)ethyl)pyrazol-4-yl]-N-[6-amino-3-(cyclopropylmethyl)-1-methyl-2,4-dioxo(1,3-dihydropyrimidin-5-yl)]carboxamide;
N-[6-amino-3-(cyclopropylmethyl)-1-ethyl-2,4-dioxo(1,3-dihydropyrimidin-5-yl)](1-{[6-(trifluoromethyl)(3-pyridyl)]methyl}pyrazol-4-yl)carboxamide;
N-[6-amino-3-propyl)-1-ethyl-2,4-dioxo(1,3-dihydropyrimidin-5-yl)](1-{(2-pyridyl)]methyl}pyrazol-4-yl)carboxamide;
N-[6-amino-3-(2-methylpropyl)-1-methyl-2,4-dioxo(1,3-dihydropyrimidin-5-yl)][1-benzylpyrazol-4-yl]carboxamide;
N-[6-amino-3-(2-methylpropyl)-1methyl-2,4-dioxo(1,3-dihydropyrimidin-5-yl)][1-{[3-fluorophenyl]methyl}pyrazol-4-yl]carboxamide;
N-[6-amino-3-(2-methylpropyl)-1-ethyl-2,4-dioxo(1,3-dihydropyrimidin-5-yl)][1-{[3-fluorophenyl]methyl}pyrazol-4-yl]carboxamide;
N-[6-amino-3-(2-methylpropyl)-1-methyl-2,4-dioxo(1,3-dihydropyrimidin-5-yl)][1-{[3-(trifluoromethyl)phenyl]methyl}pyrazol-4-yl]carboxamide; and
N-[6-amino-3-(2-methylpropyl)-1-ethyl-2,4-dioxo(1,3-dihydropyrimidin-5-yl)](1-{[6-(trifluoromethyl)(3-pyridyl)]methyl}pyrazol-4-yl)carboxamide.

### C. Preparation of a Compound of Formula (2) varying R¹ and R²

Similarly, following the procedure of Example 7A, but optionally replacing 5,6-diamino-1-ethyl-3-propyl-1,3-dihydropyrimidine-2,4-dione with other compounds of formula (2), and optionally replacing 1-{[3-(trifluoromethyl)phenyl]methyl}pyrazole-4-carboxylic acid with other compounds of formula Z-Y-X-CO₂H, other compounds of formula (3) are prepared.

### EXAMPLE 8

### Preparation of a Compound of Formula I

### A. Preparation of a Compound of Formula I in which R¹ is n-Propyl, R² is Ethyl, X is 1,4-Pyrazolyl, Y is Methylene, and Z is 3-Trifluoromethylphenyl

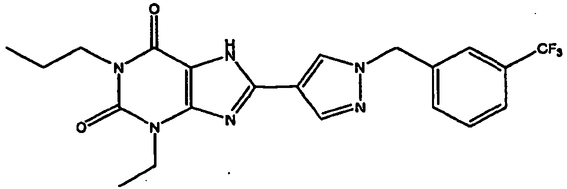

A mixture of N-(6-amino-1-ethyl-2,4-dioxo-3-propyl(1,3-dihydropyrimidin-5-yl))(1-{[3-(trifluoromethyl)phenyl]methyl}pyrazol-3-yl)carboxamide (80 mg, 0.17 mmol), 10% aqueous sodium hydroxide (5 ml), and methanol (5 ml) was stirred at 100°C for 2 hours. The mixture was cooled, methanol removed under reduced pressure, and the residue diluted with water and acidified with hydrochloric acid. The precipitate was filtered off, washed with water, then methanol, to provide 3-methyl-1-propyl-8-(1-{[3-(trifluoromethyl)phenyl]methyl}pyrazol-4-yl)-1,3,7-trihydropurine-2,6-dione, a compound of Formula I.
¹H-NMR (DMSO-d6) δ 8.57 (s, 1H), 8.15 (s, 1H), 7.60-7.75 (m, 4H), 5.54 (s, 2H), 4.05-4.50 (m, 2H), 3.87-3.91 (m, 2H), 1.55-1.64 (m, 2H), 1.25 (t, 3H, J = 7.03 Hz), 0.90 (t, 3H, J = 7.42 Hz); MS m/z 447.2 (M⁺).

### B. Preparation of a Compound of Formula I, varying R¹,_R², X, Y, and Z

Similarly, following the procedure of Example 8A, but replacing N-(6-amino-1-ethyl-2,4-dioxo-3 propyl(1,3-dihydropyrimidin-5-yl))(1-{[3-(trifluoromethyl)phenyl]-methyl}pyrazol-3-yl)carboxamide with other compounds of formula (3), the following compounds of Formula I were prepared:
1-cyclopropylmethyl-3-methyl-8-[1-(phenylmethyl)pyrazol-4-yl]-1,3,7-trihydropurine-2,6-dione;
1-cyclopropylmethyl-3-methyl-8-{1-[(3-trifluoromethylphenyl)methyl]pyrazol-4-yl}-1,3,7-trihydropurine-2,6-dione;
1-cyclopropylmethyl-3-ethyl-8-{1-[(3-trifluoromethylphenyl)methyl]pyrazol-4-yl}-1,3,7-trihydropurine-2,6-dione;
1-cyclopropylmethyl-3-methyl-8- {1-[(3-fluorophenyl)methyl]pyrazol-4-yl}-1,3,7-trihydropurine-2,6-dione;
1-cyclopropylmelhyl-3-ethyl-8-{1-[(3-fluorophenyl)methyl]pyrazol-4-yl}-1,3,7-trihydropurine-2,6-dione;
1-cyclopropylmethyl-3-ethyl-8-(1-{[6-(trifluoromethyl)(3-pyridyl)]methyl}pyrazol-4-yl)-1,3,7-trihydropurine-2,6-dione;
3-({4-[1-(cyclopropylmethyl)-3-methyl-2,6-dioxo-1,3,7-trihydropurin-8-yl]pyrazolyl} methyl)benzenecarbonitrile;
8-[1-(2-(1H-1,2,3,4-tetrazol-5-yl)ethyl)pyrazol-4-yl]-3-methyl-1-cyclopropylmethyl-1,3,7-trihydropurine-2,6-dione;
1-(2-methylpropyl)-3-methyl-8-[1-benzylpyrazol-4-yl]-1,3,7-trihydropurine-2,6-dione;
1-(2-methylpropyl)-3-ethyl-8-{1-[(3-fluorophenyl)methyl]pyrazol-4-yl}-1,3,7-trihydropurine-2,6-dione;
1-(2-methylpropyl)-3-methyl-8-{1-[(3-trifluoromethylphenyl)methyl]pyrazol-4-yl}-1,3,7-trihydropurine-2,6-dione;
1-(2-methylpropyl)-3-methyl-8-{1-[(3-fluorophenyl)methyl]pyrazol-4-yl}-1,3,7-trihydropurine-2,6-dione;
3-ethyl-1-(2-methylpropyl)-8-(1-{[6-(trifluoromethyl)(3-pyridyl)]methyl}pyrazol-4-yl)-1,3,7-trihydropurine-2,6-dione;
1-ethyl-3-methyl-8-{1-[(3-fluorophenyl)methyl]pyrazol-4-yl}-1,3,7-trihydropurine-2,6-dione; and
3-ethyl-1-propyl-8-[1-(2-pyridylmethyl)pyrazol-4-yl]-1,3,7-trihydropurine-2,6-dione.

### C. Preparation of a Compound of Formula I, varying R¹,R², X, Y, and Z

Similarly, following the procedure of Example 8A, but replacing N-(6-amino-1-ethyl-2,4-dioxo-3-propyl(1,3-dihydropyrimidin-5-yl))(1-{[3-(trifluoromethyl)phenyl]-methyl}pyrazol-3-yl)carboxamide with other compounds of formula (3), other compounds of Formula I are prepared.

### EXAMPLE 9

### Preparation of a Compound of Formula (10)

### A. Preparation of a Compound of Formula (10) in which R¹ is n-Propyl

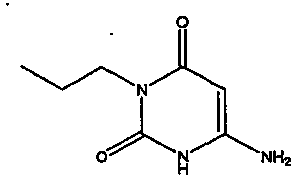

A mixture of 6-aminouracil (5.08 mg, 40 mmol), hexamethyldisilazane (50 ml), and ammonium sulfate (260 mg, 1.96 mmol) was refluxed for 12 hours. After cooling, the solid was filtered off, and solvent was removed from the filtrate under reduced pressure to provide the trimethylsilylated derivative of 6-aminouracil.

The product was dissolved in toluene (1.5 ml), and iodopropane (7.8 ml, 80 mmol) and heated in an oil bath at 120°C for 2 hours. The reaction mixture was then cooled to 0°C, and saturated aqueous sodium bicarbonate added slowly. The resulting precipitate was filtered off, and washed sequentially with water, toluene, and ether, to provide 6-amino-3,-propyl-1,3-dihydropyrimidine-2,4-dione, a compound of formula (10), which was used in the next reaction with no further purification.
¹H-NMR (DMSO-d6) δ 10.34 (s, 1H), 6.16 (s, 2H), 4.54 (s, 1H), 3.57-3.62 (m, 2H), 1.41-1.51 (m, 2H), 0.80 (t, 3H, J = 7.43 Hz).

### B. Preparation of a Compound of Formula (10), varying R¹

Similarly, following the procedure of Example 9A, but replacing iodopropane with other alkyl halides of formula R¹Hal, other compounds of formula (10) are prepared, including:
6-amino-3-cyclopropylmethyl-1,3-dihydropyrimidine-2,4-dione; and
6-amino-3-(2-methylpropyl)-1,3-dihydropyrimidine-2,4-dione.

### EXAMPLE 10

### Preparation of a Compound of Formula (11)

### A. Preparation of a Compound of Formula (11) in which R¹ is n-Propyl

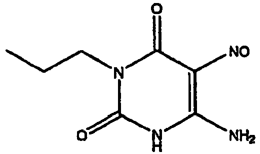

To a solution of 6-amino-3-propyl-1,3-dihydropyrimidine-2,4-dione (5.6 g) in a mixture of 50% acetic acid/water (160 ml) at 70°C was added sodium nitrite (4.5 g) in portions over a period of 15 minutes. The mixture was stirred at 70°C for 45 minutes, then the reaction mixture concentrated to a low volume under reduced pressure. The solid was filtered off, and washed with water, to provide 6-amino-5-nitroso-3-propyl-1,3-dihydropyrimidine-2,4-dione, a compound of formula (11).
¹H-NMR (DMSO-d6) δ 11.42 (s, 1H), 7.98 (s, 1H), 3.77-3.81 (m, 2H), 3.33 (s, 1H), 1.55-1.64 (m, 2H), 0.89 (t, 3H, J = 7.43 Hz); MS *m*/*z* 198.78 (M⁺), 220.78 (M⁺ +Na)

### B. Preparation of a Compound of Formula (11), varying R¹

Similarly, following the procedure of Example 10A, but replacing 6-amino-3-propyl-1,3-dihydropyrimidine-2,4-dione with other compounds of formula (10), other compounds of formula (11) are prepared, including:
6-amino-5-nitroso-3-cyclopropylmethyl-1,3-dihydropyrimidine-2,4-dione; and
6-amino-5-nitroso-3-(2-methylpropyl)-1,3-dihydropyrimidine-2,4-dione.

### EXAMPLE 11

### Preparation of a Compound of Formula (12)

### A. Preparation of a Compound of Formula (12) in which R¹ is n-Propyl

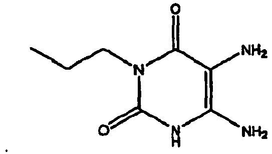

To a solution of 6-amino-5-nitroso-3-propyl-1,3-dihydropyrimidine-2,4-dione (5.4 g, 27 mmol) in 12.5% aqueous ammonia (135 ml) at 70°C was added sodium dithionite (Na₂S₂O₄, 9.45 g 54 mmol) in portions over 15 minutes, and the mixture was stirred for 20 minutes. The solution was concentrated under reduced pressure, cooled to 5°C, the precipitate filtered off, and washed with cold water, to provide 5,6-diamino-3-propyl-1,3-dihydropyrimidine-2,4-dione, a compound of formula (12).
¹H-NMR (DMSO-d6) δ 0.81 (t, 3H, J = 7.43 Hz), 1.43-1.52 (m, 2H), 3.63-3.67 (m, 2H), 5.56 (s, 2H); MS *m*/*z* 184.95 (M⁺), 206.96 (M⁺ +Na)

### B. Preparation of a Compound of Formula (12), varying R¹

Similarly, following the procedure of Example 11A, but replacing 6-amino-3-propyl-1,3-dihydropyrimidine-2,4-dione with other compounds of formula (11), other compounds of formula (12) are prepared, including:
5,6-diamino-3-cyclopropylmethyl-1,3-dihydropyrimidine-2,4-dione; and
5,6-diamino-3-(2-methylpropyl)-1,3-dihydropyrimidine-2,4-dione.

### EXAMPLE 12

### Preparation of a Compound of Formula (13)

### A. Preparation of a Compound of Formula (13) in which R¹ is n-Propyl X is 1.4-Pyrazolyl, Y is Methylene, and Z is 3-Trifluoromethylphenyl

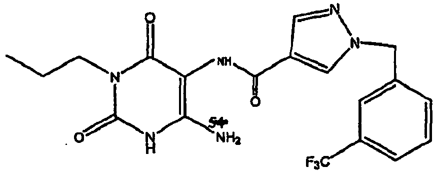

To a mixture of 5,6-diamino-3-propyl-1,3-dihydropyrimidine-2,4-dione (2.3 g, 126 mmol) and 1-{[3-(trifluoromethyl)phenyl]methyl}pyrazole-4-carboxylic acid (3.79 g, 14 mmol) in methanol (50 ml) was added 1-(3-dimethylaminopropyl)-3-ethylcarbodiimide hydrochloride (2.67 g, 14 mmol), and the reaction mixture was stirred for 3 days at room temperature (although less time is acceptable). The precipitate was filtered off, and was washed sequentially with water, and methanol. The product was dried under vacuum to provide N-(6-amino-2,4-dioxo-3-propyl(1,3-dihydropyrimidin-5-yl))(1-{[3-(trifluoromethyl)phenyl]methyl}pyrazol-4-yl)carboxamide, a compound of formula (13).
¹H-NMR (DMSO-d6) δ 10.44 (s, 1H), 8.56 (s, 1H), 8.37 (s, 1H), 8.00 (s, 1H), 7.56-7.71 (m, 3H), 6.02 (s, 1H), 5.49 (s, 2H), 3.62-3.66 (m, 2H), 1.44-1.53 (m, 2H), 0.82 (t, , 3H, J = 7.43 Hz); MS m/z 458.92 (M⁺ +Na).

### B. Alternative Preparation of a Compound of Formula (13) in which R¹ is n-Propyl X is 1,4 Pyrazolyl, Y is Methylene, and Z is 3-Trifluoromethylphenyl

A solution of 1-{[3-(trifluoromethyl)phenyl]methyl}pyrazole-4-carboxylic acid (1 g, 3.7 mmol) in thionyl chloride (1 ml) was heated at 70°C for 4 hours. Excess thionyl chloride was distilled off, and the residue treated with methylene chloride/hexanes. The solvent was removed under reduced pressure, and the residue dissolved in acetonitrile. This solution was added to a suspension of 5,6-diamino-3-propyl-1,3-dihydropyrimidine-2,4-dione (2.3 g, 126 mmol) and triethylamine (1 ml) in acetonitrile (20 ml) at 0°C, and stirred for 16 hours. The reaction mixture was quenched with water (5 ml), acidified with hydrochloric acid, stirred for 30 minutes, and the precipitate filtered off. The product was washed with ether, to provide N-(6-amino-2,4-dioxo-3-propyl(1,3-dihydropyrimidin-5-yl))(1-{[3-(trifluoromethyl)phenyl]methyl}pyrazol-4-yl)carboxamide, a compound of formula (13).

### C. Preparation of a Compound of Formula (13), varying R¹, X, Y, and Z

Similarly, following the procedure of Example 12A or 12B, but optionally replacing 6-amino-3-propyl-1,3-dihydropyrimidine-2,4-dione with other compounds of formula (12), and optionally replacing 1-{[3-(trifluoromethyl)phenyl]methyl}pyrazole-4-carboxylic acid with other compounds of formula Z-Y-X-CO₂H, other compounds of formula (13) are prepared, including:

N-(6-amino-2,4-dioxo-3-cyclopropylmethyl(1,3-dihydropyrimidin-5-yl))(1-{[3-(trifluoromethyl)phenyl]methyl}pyrazol-4-yl)carboxamide;

N-(6-amino-2,4-dioxo-3-(2-methylpropyl)(1,3-dihydropyrimidin-5-yl))(1-{[3-(trifluoromethyl)phenyl]methyl}pyrazol-4-yl)carboxamide;

N-(6-amino-2,4-dioxo-3-propyl(1,3-dihydropyrimidin-5-yl))(1-{[3-fluorophenyl]methyl}pyrazol-4-yl)carboxamide;

N-(6-amino-2,4-dioxo-3-cyclopropylmethyl(1,3-dihydropyrimidin-5-yl))(1-{[3-fluorophenyl]methyl}pyrazol-4-yl)carboxamide;

N-(6-amino-2,4-dioxo-3-(2-methylpropyl)(1,3-dihydropyrimidin-5-yl))(1-{[3-fluorophenyl]methyl}pyrazol-4-yl)carboxamide;

N-(6-amino-2,4-dioxo-3-propyl(1,3-dihydropyrimidin-5-yl))(1-[1-benzyl]pyrazol-4-yl)carboxamide;

N-(6-amino-2,4-dioxo-3-cyclopropylmethyl(1,3-dihydropyrimidin-5-yl))(1-[1-benzyl]pyrazol-4-yl)carboxamide;

N-(6-amino-2,4-dioxo-3-(2-methylpropyl)(1,3-dihydropyrimidin-5-yl))(1-[1-benzyl]pyrazol-4-yl)carboxamide;

N-(6-amino-2,4-dioxo-3-propyl(1,3-dihydropyimidin-5-yl))(1-{[3-cyanophenyl]methyl}pyrazol-4-yl)carboxamide;

N-(6-amino-2,4-dioxo-3-cyclopropylmethyl(1,3-dihydropyrimidin-5-yl))(1-{[3-cyanophenyl]methyl}pyrazol-4-yl)carboxamide;

N-(6-amino-2,4-dioxo-3-(2-methylpropyl)(1,3-dihydropyrimidin-5-yl))(1-{[3-cyanophenyl]methyl}pyrazol-4-yl)carboxamide;

N-(6-amino-2,4-dioxo-3-propyl(1,3-dihydropyrimidin-5-yl))(1-({1-(2-(1H-1,2,3,4-tetrazol-5-yl)ethyl)pyrazol-4-yl}carboxamide;

N-(6-amino-2,4-dioxo-3-cyclopropylmethyl(1,3-dihydropyrimidin-5-yl))(1-{[1-(2-(1H-1,2,3,4-tetrazol-5-yl)ethyl)pyrazol-4-yl)carboxamide;

N-(6-amino-2,4-dioxo-3-(2-methylpropyl)(1,3-dihydropyrimidin-5-yl))(1-{[1-(2-(1H-1,2,3,4-tetrazol-5-yl)ethyl)pyrazol-4-yl)carboxamide;

N-(6-amino-2,4-dioxo-3-propyl(1,3-dihydropyrimidin-5-yl))(1-{[6-(trifluoromethyl)(3-pyridyl)]methyl}pyrazol-4-yl)carboxamide;

N-(6-amino-2,4-dioxo-3-cyclopropylmethyl(1,3-dihydropyrimidin-5-yl))(1-{[6-(trifluoromethyl)(3-pyridyl)]methyl}pyrazol-4-yl)carboxamide; and

N-(6-amino-2,4-dioxo-3-(2-methylpropyl)(1,3-dihydropyrimidin-5-yl))(1-{[6-(trifluoromethyl)(3-pyridyl)]methyl}pyrazol-4-yl)carboxamide.

### EXAMPLE 13

### Preparation of a Compound of Formula (3)

### A. Preparation of a Compound of Formula (3) in which R¹ is n-Propyl, R² is Ethyl, X is 1,4-Pyrazolyl, Y is Methylene, and Z is 3-Trifluoromethylphenyl 1

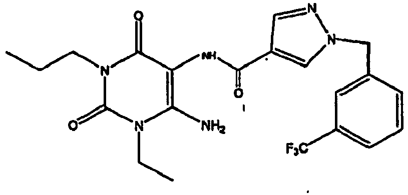

A mixture of a solution of N-(6-amino-2,4-dioxo-3-propyl(1,3-dihydropyrimidin-5-yl))(1-{[3-(trifluoromethyl)-phenyl]methyl}pyrazol-3-yl)carboxamide (872 mg, 2 mmol) in dimethylformamide (10 ml), potassium carbonate (552 mg, 4 mmol) and ethyl iodide (0.24 ml, 3 mmol) was stirred at room temperature overnight. The reaction mixture was filtered, and the solvent was evaporated from the filtrate under reduced pressure. The residue was stirred with water for two hours at room temperature, and the precipitate filtered off, washed with water, and then dissolved in methanol. The solvent was then removed under reduced pressure to provide N-(6-amino-1-ethyl-2,4-dioxo-3-propyl(1,3-dihydropyrimidin-5-yl))(1-{[3-(trifluoromethyl)phenyl]methyl}pyrazol-4-yl)carboxamide, a compound of formula (3).
¹H-NMR (DMSO-d6): δ 8.58 (s,1H), 8.39 (s, 1H), 8.01 (s, 1H), 7.72 - 7.50 (m, 4H), 6.71 (s, 2H), 5.51 (s, 2H), 4.0 - 3.82 (m, 2H), 3.77 - 3.65 (m, 2H), 1.60 -1.50 (m, 2H), 1.13 (t, 3H, J = 6.8 Hz), 0.84 (t, 3H, J = 7.2 Hz); MS *m*/*z* 462.9 (M⁻)

### B. Preparation of a Compound of Formula (13), varying R¹, X, Y, and Z

Similarly, following the procedure of Example 13A, but replacing N-(6-amino-2,4-dioxo-3-propyl(1,3-dihydropyrimidin-5-yl))(1-{[3-(trifluoromethyl)-phenyl]methyl}pyrazol-3-yl)carboxamide with other compounds of formula (13), other compounds of formula (3) are prepared, including:

N-(6-amino-1-methyl-2,4-dioxo-3-propyl(1,3-dihydropyrimidin-5-yl))(1-{[3-(trifluoromethyl)phenyl]methyl}-pyrazol-4-yl)carboxamide;

N-(6-amino-1-methyl-2,4-dioxo-3-cyclopropylmethyl(1,3-dihydropyrimidin-5-yl))(1-{[3-(trifluoromethyl)phenyl]methyl} pyrazol-4-yl)carboxamide;

N-(6-amino-1-ethyl-2,4-dioxo-3-cyclopropylmethyl(1,3-dihydropyrimidin-5-yl))(1- {[3-(trifluoromethyl)phenyl]methyl}-pyrazol-4-yl)carboxamide;

N-(6-amino-1-methyl-2,4-dioxo-3-ethyl(1,3-dihydropyrimidin-5-yl))(1-{[3-fluorophenyl]methyl}-pyrazol-4-yl)carboxamide;

N-(6-amino-1-methyl-2,4-dioxo-3-cyclopropylmethyl(1,3-dihydropyrimidin-5-yl))(1-{[3-fluorophenyl]methyl}-pyrazol-4-yl)carboxamide;

N-(6-amino-1-ethyl-2,4-dioxo-3-cyclopropylmethyl(1,3-dihydropyrimidin-5-yl))(1-{[3-fluorophenyl]methyl}-pyrazol-4-yl)carboxamide;

N-[6-amino-3-(cyclopropylmethyl)-1-methyl-2,4-dioxo(1,3-dihydropyrimidin-5-yl)][1-benzylpyrazol-4-yl]carboxamide;

N-(6-amino-1-methyl-2,4-dioxo-3-cyclopropylmethyl(1,3-dihydropyrimidin-5-yl))(1-{[3-cyanophenyl]methyl}-pyrazol-4-yl)carboxamide;

[1-(2-(1H-1,2,3,4-tetrazol-5-yl)ethyl)pyrazol-4-yl]-N-[6-amino-3-(cyclopropylmethyl)-1-methyl-2,4-dioxo(1,3-dihydropyrimidin-5-yl)]carboxamide;

N-[6-amino-3-(cyclopropylmethyl)-1-ethyl-2,4-dioxo(1,3-dihydropyrimidin-5-yl)](1-{[6-(trifluoromethyl)(3-pyridyl)]methyl}pyrazol-4-yl)carboxamide;

N-[6-amino-3-propyl)-1-ethyl-2,4-dioxo(1,3-dihydropyrimidin-5-yl)](1-{(2-pyridyl)]methyl}pyrazol-4-yl)carboxamide; .

N-[6-amino-3-(2-methylpropyl)-1-methyl-2,4-dioxo(1,3-dihydropyrimidin 5-yl)][1-benzylpyrazol-4-yl]carboxamide;

N-[6-amino-3-(2-methylpropyl)-1-methyl-2,4-dioxo(1,3-dihydropyrimidin-5-yl)][1-{[3-fluorophenyl]methyl}pyrazol-4-yl]carboxamide;

N-[6-amino-3-(2-methylpropyl)-1-ethyl-2,4-dioxo(1,3-dihydropyrimidin-5-yl)][1-{[3-fluorophenyl]methyl}pyrazol-4-yl]carboxamide;

N-[6-amino-3-(2-methylpropyl)-1-methyl-2,4-dioxo(1,3-dihydropyrimidin-5-yl)][1-{[3-(trifluoromethyl)phenyl]methyl}pyrazol-4-yl]carboxamide; and

N-[6-amino-3-(2-methylpropyl)-1-ethyl-2,4-dioxo(1,3-dihydropyrimidin 5-yl)](1-{[6-(trifluoromethyl)(3-pyridyl)]methyl}pyrazol-4-yl)carboxamide.

### EXAMPLE 14

### Preparation of a Compound of Formula I

### A. Preparation of a Compound of Formula I in which R¹ is n-Propyl, R² is Ethyl, X is 1,4-Pyrazolyl, Y is Methylene, and Z is 3-Trifluoromethylphenyl

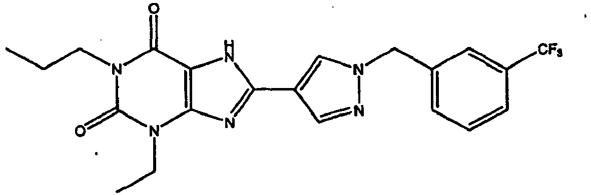

A mixture of N-(6-amino-1-ethyl-2,4-dioxo-3-propyl(1,3-dihydropyrimidin-5-yl))(1-{[3-(trifluoromethyl)phenyl]methyl}pyrazol-3-yl)carboxamide (850 mg, 2.34 mmol), 10% aqueous sodium hydroxide (10 ml), and methanol (10 ml) was stirred at 100°C for 18 hours. The mixture was cooled, methanol removed under reduced pressure, and the remaining mixture was acidified with hydrochloric acid to pH 2. The precipitate was filtered off, washed with water/methanol mixture, to provide 3-ethyl-1-propyl-8-(1-{[3-(trifluoromethyl)phenyl]methyl}pyrazol-4-yl)-1,3,7-trihydropurine-2,6-dione, a compound of Formula I.
¹H-NMR (DMSO-d6) δ 8.57 (s, 1H), 8.15 (s, 1H), 7.60-7.75 (m, 4H), 5.54 (s, 2H), 4.05-4.50 (m, 2H), 3.87-3.91 (m, 2H), 1.55-1.64 (m, 2H), 1.25 (t, 3H, J = 7.03 Hz), 0.90 (t, 3H, J = 7.42 Hz); MS *m*/*z* 447.2 (M⁺)

### B. Preparation of a Compound of Formula L varying R¹, R², X, Y, and Z

Similarly, following the procedure of Example 14A, but replacing N-(6-amino-1-ethyl-2,4-dioxo-3-propyl(1,3-dihydropyrimidin-5-yl))(1-{[3-(trifluoromethyl)phenyl]methyl}pyrazol-3-yl)carboxamide with other compounds of formula (3), other compounds of Formula I are prepared, including those listed in Example 8.

### EXAMPLE 15

### Preparation of a Compound of Formula (14)

### A. Preparation of a Compound of Formula (14) in which R² is Ethyl

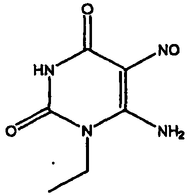

To a solution of 6-amino-1-ethyl-1,3-dihydropyrimidine-2,4-dione (5.0 g, 32.3 mmol) in a mixture of 50% acetic acid/water (50 ml) at 70°C was added sodium nitrite (4.45 g, 64.5 mmol) in portions over a period of 30 minutes. The mixture was stirred at 70°C for a further 30 minutes. The reaction mixture was cooled, and the precipitate filtered off, and washed with water, then methanol, to provide 6-amino-1-ethyl-5-nitroso-1,3-dihydropyrimidine-2,4-dione, a compound of formula (14).
¹H-NMR (DMSO-d6): δ 11.52 (s, 1H), 9.16 (s, 1H), 3.83 (q, 2H, J = 7.0 Hz), 1.11 (t, 3H, J = 7.0 Hz). MS *m*/*z* 184.8 (M⁺), 206.80 (M⁺ +Na)

### B. Preparation of a Compound of Formula (14), varying R²

Similarly, following the procedure of Example 15A, but replacing 6-amino-1-ethyl-1,3-dihydropyrimidine-2,4-dione with 6-amino-1-methyl-1,3-dihydropyrimidine-2,4-dione, 6-amino-1-methyl-5-nitroso-1,3-dihydropyrimidine-2,4-dione was prepared.

### C. Preparation of a Compound of Formula (14), varying R²

Similarly, following the procedure of Example 15A, but replacing 6-amino-1-ethyl-1,3-dihydropyrimidine-2,4-dione with other compounds of formula (5), other compounds of formula (14) are prepared.

### EXAMPLE 16

### Preparation of a Compound of Formula (15)

### A. Preparation of a Compound of Formula (15) in which R² is Ethyl

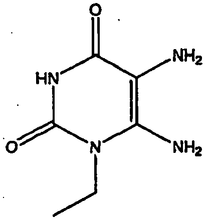

To a solution of 6-amino-1-ethyl-5-nitroso-1,3-dihydropyrimidine-2,4-dione (3.9 g, 21.2 mmol) in 14.5% aqueous ammonia (50 ml) at 50°C was added sodium dithionite (Na₂S₂O₄, 7.37 g, 42.4 mmol) in portions over 15 minutes, and the mixture was stirred for 20 minutes. The solution was concentrated under reduced pressure to a volume of 30 ml, cooled to 5°C, the precipitate filtered off, and washed with cold water, to provide 5,6-diamino-1-ethyl-1,3-dihydropyrimidine-2,4-dione, a compound of formula (15).
¹H-NMR (DMSO-d6): δ 10.58 (s, 1H), 6.18 (s, 2H), 3.83 (q, 2H, J = 7.2 Hz), 2.82 (s, 2H), 1.10 (t, 3H, J = 7.2 Hz).

### B. Preparation of a Compound of Formula (15), varving R²

Similarly, following the procedure of Example 16A, but replacing 6-amino-1-ethyl-5-nitroso-1,3-dihydropyrimidine-2,4-dione with 6-amino-1-methyl-5-nitroso-1,3-dihydropyrimidine-2,4-dione, 5,6-diamino-1-methyl-1,3-dihydropyrimidine-2,4-dione was prepared.

### C. Preparation of a Compound of Formula (15), varying R²

Similarly, following the procedure of Example 16A, but replacing 6-amino-1-ethyl-5-nitroso-1,3-dihydropyrimidine-2,4-dione with other compounds of formula (14), other compounds of formula (15) are prepared.

### EXAMPLE 17

### Preparation of a Compound of Formula (16)

### A. Preparation of a Compound of Formula (16) in which R² is Ethyl, X is 1,4-Pyrazolyl, Y is Methylene, and Z is 3-Trifluoromethylphenyl

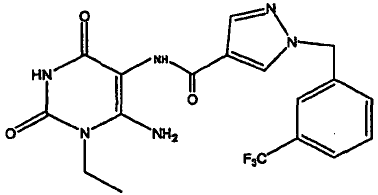

To a mixture of 5,6-diamimo-1-ethyl-1,3-dihydropyrimidine-2,4-dione (2 g, 11.76 mmol) and 1-{[3-(trifluoromethyl)phenyl]methyl}pyrazole-4-carboxylic acid (3.5 g, 12.94 mmol) in methanol (50 ml) was added 1-(3-dimethylaminopropyl)-3-ethylcarbodiimide hydrochloride (2.47 g, 12.94 mmol), and the reaction mixture was stirred for 16 hours at room temperature. Solvent was removed under reduced pressure, and the residue was washed with water and methanol. The product was dried under vacuum to provide N-(6-amino-1-ethyl-2,4-dioxo(1,3-dihydropyrimidin-5-yl))(1-{[3-(trifluoromethyl)phenyl]methyl}pyrazol-4-yl)carboxamide, a compound of formula (16).
¹H-NMR (DMSO-d6): δ 10.60 (s, 1H), 8.50 (s, 1H), 8.39 (s, 1H), 8.01 (s, 1H), 7.72-7.50 (m, 4H), 6.69 (s, 2H), 5.50 (s, 2H), 3.87 (q, 2H, J = 7.2 Hz), 1.11 (t, 3H, 7.2 Hz); MS m/z 421 (M⁻)

### B. Preparation of a Compound of Formula (16), varying R², X, Y, and Z

Similarly, following the procedure of Example 17A, but replacing 5,6-diamino-1-ethyl-1,3-dihydropyrimidine-2,4-dione with 5,6-diamino-1-methyl-1,3-dihydropyrimidine-2,4-dione, N-(6-amino-1-methyl-2,4-dioxo(1,3-dihydropyrimidin-5-yl))(1-{[3-(trifluoromethyl)phenyl]methyl}pyrazol-4-yl)carboxamide was prepared.

### C. Preparation of a Compound of Formula (16), varying R², X, Y, and Z

Similarly, following the procedure of Example 17A, but replacing 5,6-diamino-1-ethyl-1,3-dihydropyrimidine-2,4-dione with other compounds of formula (15), other compounds of formula (16) are prepared.

### EXAMPLE 18

### Preparation of a Compound of Formula (3)

### A. Preparation of a Compound of Formula (3) in which R¹ is n-Propyl, R² is Ethyl, X is 1,4-Pyrazolyl, Y is Methylene, and Z is 3-Trifluoromethylphenyl

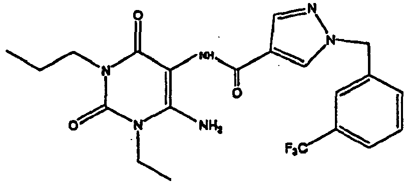

A mixture of a solution of N-(6-amino-1-ethyl-2,4-dioxo(1,3-dihydropyrimidin-5-yl))(1-{[3-(trifluoromethyl)phenyl]methyl}pyrazol-3-yl)carboxamide (1.5 g, 3.55 mmol) in dimethylformamide (30 ml), potassium carbonate (980 mg, 7.1 mmol) and propyl iodide (724 mg, 4.26 mmol) was stirred at room temperature overnight. Water was added, and the precipitate filtered off, to provide N-(6-amino-1-ethyl-2,4-dioxo-3-propyl(1,3-dihydropyrimidin-5-yl))(1-{[3-(trifluoromethyl)phenyl]methyl}pyrazol-4-yl)carboxamide, a compound of formula (3), which was used in the next reaction with no further purification.
¹H-NMR (DMSO-d6): δ 8.58 (s, 1H), 8.39 (s, 1H), 8.01 (s, 1H), 7.72 - 7.50 (m, 4H), 6.71 (s, 2H), 5.51 (s, 2H), 4.0 - 3.82 (m, 2H), 3.77 - 3.65 (m, 2H), 1.60 -1.50 (m, 2H), 1.13 (t, 3H, J = 6.8 Hz), 0.84 (t, 3H, J = 7.2 Hz); MS *m*/*z* 462.9 (M⁻)

### B. Preparation of a Compound of Formula (3), varying R¹, R², X, Y, and Z

Similarly, following the procedure of Example 18A, but replacing N-(6-amino-1-ethyl-2,4-dioxo(1,3-dihydropyrimidin-5-yl))(1-{[3-(trifluoromethyl)phenyl]-methyl}pyrazol-3-yl)carboxamide with N-(6-amino-1-methyl-2,4-dioxo(1,3-dihydropyrimidin-5-yl)), N-(6-amino-1-methyl-2,4-dioxo-3-propyl(1,3-dihydropyrimidin-5-yl))(1-{[3-(trifluoromethyl)phenyl]methyl}pyrazol-4-yl)carboxamide was prepared.

### C. Preparation of a Compound of Formule (3), varying R¹, R², X, Y, and Z

Similarly, following the procedure of Example 18A, but optionally replacing N-(6-amino-1-ethyl-2,4-dioxo(1,3-dihydropyrimidin-5-yl))(1-{[3-(trifluoromethyl)phenyl]methyl}pyrazol-3-yl)carboxamide with other compounds of formula (16), and optionally replacing propyl iodide with other compounds of formula R¹Hal, other compounds of formula (3) are prepared.

### EXAMPLE 19

### Preparation of a Compound of Formula I

### A. Preparation of a Compound of Formula I in which R¹ is n-Propyl, R² is Ethyl, X is 1,4-Pyrazolyl, Y is Methylene, and Z is 3-Trifluoromethylphenyl

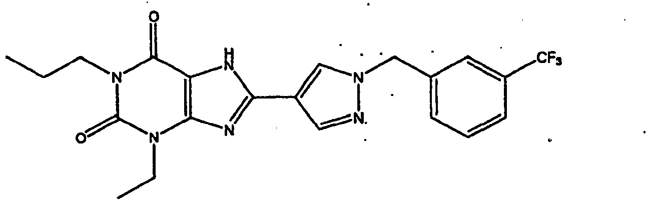

A mixture of N-(6-amino-1-ethyl-2,4-dioxo-3-propyl(1,3-dihydropyrimidin-5-yl))(1-{[3-(trifluoromethyl)phenyl]methyl}pyrazol-3-yl)carboxamide (300 mg, 464 mmol), 20% aqueous sodium hydroxide (5 ml), and methanol (10 ml) was stirred at 80°C for 3 hours. The mixture was cooled, methanol removed under reduced pressure, and the remaining mixture was acidified with hydrochloric acid to pH 2. The precipitate was filtered off, washed with water and methanol, to provide 3-ethyl-1-propyl-8-(1-{[3-(trifluoromethyl)phenyl]methyl}pyrazol-4-yl)-1,3,7-trihydropurine-2,6-dione, a compound of Formula I.
¹H-NMR (DMSO-d6) δ 8.57 (s, 1H), 8.15 (s, 1H), 7.60-7.75 (m, 4H), 5.54 (s, 2H), 4.05-4.50 (m, 2H), 3.87-3.91 (m, 2H), 1.55-1.64 (m, 2H), 1.25 (t, 3H, J = 7.03 Hz), 0.90 (t, 3H, J = 7.42 Hz); MS *m*/*z* 447.2 (M⁺)

### EXAMPLE 20

### CHARACTERIZATION OF A_{2B} ANTAGONISTS

### Radioligand binding for A_{2B} adenosine receptor

Human A_{2B} adenosine receptor cDNA was stably transfected into HEK-293 cells (referred to as HEK-A2B cells). Monolayers of HEK-A2B cells were washed with PBS once and harvested in a buffer containing 10 mM HEPES (pH 7.4),10 mM EDTA and protease inhibitors. These cells were homogenized in polytron for 1 minute at setting 4 and centrifuged at 29000 g for 15 minutes at 4°C. The cell pellets were washed once with a buffer containing 10 mM HEPES (pH 7.4), 1 mM EDTA and protease inhibitors, and were resuspended in the same buffer supplemented with 10% sucrose. Frozen aliquots were kept at -80°C.

Competition assays were started by mixing 14 nM ³H-ZM214385 (Tocris Cookson) with various concentrations of test compounds and 50 µg membrane proteins in TE buffer (50 mM Tris and 1 mM EDTA) supplemented with 1 Unit/mL adenosine deaminase. The assays were incubated for 90 minutes, stopped by filtration using Packard Harvester and washed four times with ice-cold TM buffer (10 mM Tris, 1 mM MgCl2, pH 7.4). Non specific binding was determined in the presence of 10 µM ZM214385. The affinities of compounds (i.e. Ki values) were calculated using GrraphPad software.

### Radioligand binding for other adenosine receptors

Human A₁, A_{2A}, A₃ adenosine receptor cDNAs were stably transfected into either CHO or HEK-293 cells (referred to as CHO-A₁, HEK-A_{2A}, CHO-A₃). Membranes were prepared from these cells using the same protocol as described above. Competition assays were started by mixing 0.5 nM ³H-CPX (for CHO-A₁), 2 nM ³H-ZM214385 (HEK-A_{2A}) or 0.1 nM ¹²⁵I-AB-MECA (CHO-A₃) with various concentrations of test compounds and the perspective membranes in TE buffer (50 mM Tris and 1 mM EDTA of CHO-A₁ and HEK-A_{2A}) or TEM buffer (50 mM Tris, 1 mM EDTA and 10 mM MgCl₂ for CHO-A₃) supplemented with 1 Unit/mL adenosine deaminase. The assays were incubated for 90 minutes, stopped by filtration using Packard Harvester and washed four times with ice-cold TM buffer (10 mM Tris, 1 mM MgCl₂, pH 7.4). Non specific binding was determined in the presence of 1 µM CPX (CHO-A₁), 1 µM ZM214385 (HEK-A_{2A}) and 1 µM IB-MECA (CHO-A₃). The affinities of compounds (i.e. Ki values) were calculated using GraphPad^{™} software.

### cAMP measurements

Monolayer of transfected cells were collected in PBS containing 5 mM EDTA. Cells were washed once with DMEM and resuspended in DMEM containing 1 Unit/mL adenosine deaminase at a density of 100,000-500,000 cells/ml. 100 µl of the cell suspension was mixed with 25 µl containing various agonists and/or antagonists and the reaction was kept at 37°C for 15 minutes. At the end of 15 minutes, 125 µl 0.2 N HCl was added to stop the reaction. Cells were centrifuged for 10 minutes at 1000 rpm. 100 µl of the supernatant was removed and acetylated. The concentrations of cAMP in the supernatants were measured using the direct cAMP assay from Assay Design. A_{2A} and A_{2B} adenosine receptors are coupled to Gs proteins and thus agonists for A_{2A} adenosine receptor (such as CGS21680) or for A_{2B} adenosine receptor (such as NECA) increase the cAMP accumulations whereas the antagonists to these receptors prevent the increase in cAMP accumulations-induced by the agonists. A₁ and A₃ adenosine receptors are coupled to Gi proteins and thus agonists for A₁ adenosine receptor (such as CPA) or for A₃ Adenosine receptor (such as IB-MECA) inhibit the increase in cAMP accumulations-induced by forskolin. Antagonists to A₁ and A₃ receptors prevent the inhibition in cAMP accumulations.

### EXAMPLE 21

### EFFECT OF A_{2B} ANTAGONIST ON WOUND HEALING IN MOUSE MODEL

Groups of ICR derived male mice (weighing 24 ±2 g) of 5 each were used. During testing period, the animals were single-housed in each cage. Under hexobarbital (90 mg/kg, IP) anesthesia, the shoulder and back region of each animal was shaved. A sharp punch (ID 12 mm) was applied to remove the skin including *panniculus carnosus* and adherent tissues. The wound area, traced onto clear plastic sheets on days 1, 3, 5, 7, 9 and 11, was measured by use of an Image Analyzer (Life Science Resources Vista, Version 3.0). Test substances were administered topically immediately following wound injury once daily for a total of 10 consecutive days. The closure of the wound (%) and wound half-closure time (CT₅₀) were determined by linear regression using Graph-Pad Prism^{™} (Graph Pad Software USA) and unpaired Student's t test was applied for comparison between treated and vehicle groups at each measurement time point on days 3, 5, 7, 9 and 11. Differences are considered of statistical significance at P<0.05 level.

Table 1 presents test data obtained for the compound 3-ethyl-1-propyl-8-(1-{[3-(trifluoromethyl)phenyl]methyl}pyrazol-4-yl)-1,3,7-trihydropurine-2,6-dione using the mouse model discussed above. Table 2 presents test data obtained using 1,3-dipropyl-8-(1-{[6-(trifluoromethyl)(3-pyridyl)]methyl}pyrazol-4-yl)-1,3,7-trihydropurine-2,6-dine. In each case, the vehicle used for comparison was 1.5% carboxymethylcellulose in phosphate buffered saline at pH 7.4

**Table 1**

| **TREATMENT** | **DOSE** | | **PERCENT WOUND CLOSURE** | | | | | | | | | | | **CT₅₀ DAYS** |
|---|---|---|---|---|---|---|---|---|---|---|---|---|---|---|
| | | | **Day 1** | **Day 2** | **Day 3** | **Day 4** | **Day 5** | **Day 6** | **Day 7** | **Day 8** | **Day 9** | **Day 10** | **Day 11** | |
| Vehicle | 20 | X | 0 | 21.7 | 31.5 | 44.1 | 48.0 | 52.8 | 60.1 | 68.8 | 73.2 | 77.7 | 80.3 | 5.9 |
| | µl/mouse | SEM | | 3.2 | 3.0 | 2.1 | 3.1 | 2.9 | 2.7 | 1.6 | 1.5 | 0.8 | 1.6 | 0.3 |
| 3-ethyl-1-propyl-8-(1-{[3-(trifluoromethyl)phenyl]methyl}pyrazol-4-yl)-1,3,7-trihydropurine-2,6-dione | 2 | X | 0 | 35.6* | 46.5* | 57.4* | 62.4 | 68.3* | 73.3* | 78.8* | 82.2* | 89.2* | 91.6* | 4.4* |
| | µg/mouse | SEM | | 4.1 | 3.5 | 2.5 | 1.3 | 1.3 | 1.0 | 12 | 1.5 | 2.8 | 2.4 | 0.2 |
| | 0.5 | X | 0 | 43.2* | 51.2* | 56.0* | 58.8* | 64.3* | 72.5* | 78.2* | 82.5* | 83.1* | 87.1* | 4.3* |
| | µg/mouse | SEM | | 3.1 | 3.4 | 1.8 | 2.0 | 1.1 | 0.9 | 1.6 | 1.2 | 1.3 | 0.8 | 0.2 |

| | | | | | | | | | | | | | | |
|---|---|---|---|---|---|---|---|---|---|---|---|---|---|---|
| The percent wound closure and half closure time (CF₅₀) were determined and unpaired Student's *t* test was used tor the comparison of data obtained between treated and vehicle groups (n = 5 each). **p*<0.05, statistically significant. | | | | | | | | | | | | | | |

**Table 2**

| **TREATMENT** | **DOSE** | | **PERCENT WOUND CLOSURE** | | | | | **CT₅₀ DAYS** |
|---|---|---|---|---|---|---|---|---|
| | | | **Day 3** | **Day 5** | **Day 7** | **Day 9** | **Day 11** | |
| Vehicle | 20 | X | 28.4 | 44.3 | 54.2 | 66.4 | 72.4 | 6.8 |
| | µl/mouse | SEM | 2.2 | 2.1 | 2.9 | 2.2 | 0.7 | 0.2 |
| 1,3-dipropyl-8-(1-{[6-trifluoromethyl)(3-pyridyl)]methyl}pyrazol-4-yl)-1,3,7-trihydropurine-2,6-dione | 0.5 | X | 41.9* | 53.5* | 65.8* | 77.1* | 85.3* | 5.5* |
| | µg/mouse | SEM | 1.4 | 2.7 | 1.9 | 1.7 | 1.1 | 0.1 |

| | | | | | | | | |
|---|---|---|---|---|---|---|---|---|
| The percent wound closure and halt closure time (CT₅₀) were determined and unpaired Student's *t* test was used for the comparison or data obtained between treated and vehicle groups (n = 5 each). **p*<0.05, statistically significant. | | | | | | | | |

### EXAMPLE 22

### EFFECT OF A_{2B} ANTAGONIST ON WOUND HEALING IN PIG MODEL

Pig skin heals most like human skin and therefore testing on this animal provides an optimal paradigm to study cutaneous repair mechanisms. In this Example, the effect of an A_{2B} antagonist on wound healing was tested in three pigs. PDGF (REGRANEX^{®} (becaplermin) Gel 0.01%, Ortho-McNeil Pharmaceutical, Inc., Raritan, NJ) was used as a positive control. The vehicle for drug delivery was 1.5% methylcellulose gel (KY gel). Control wounds received the vehicle alone. 3 different dosages of the A_{2B} antagonist 3-ethyl-1-propyl-8-(1-{[3-(trifluoromethyl)phenyl]methyl}pyrazol-4-yl)-1,3,7-trihydropurine-2,6-dione, 1 µg/40 µl, 4 µg/40 µl, 1 µg/40 µl, and 20 µg/40 µl were tested.

Method: Three Yorkshire pig (-75-90 lbs.) were used. On the day of surgery, a series of full thickness excisions were created alongside the paravertebral region on one side of the pig. Pigs were sacrificed at the end of 10 days and all wounds were removed for histological and immunohistochemical examinations.

### Effects on general stimulation_of_healing within the Dermis (Granulation Tissue)

The A_{2B} adenosine receptor antagonist stimulated the total granulation tissue in a dose-dependent fashion. There is a statistical difference between the placebo formulation and the highest dose 20 µg/40 µl (*p* = 0.035), between the lowest dose 1 µg/40 µl and the highest dose 20 µg/40 µl (*p* = 0.047) and between the placebo formulation and the positive control PDGF (*p* =0.034). Thus, our data indicates that topical dosing with an A_{2B} adenosine receptor antagonist produces a desirable biological response in the porcine model.

## Claims

1. Use of an A_{2B} receptor antagonist having the structure of Formula I or Formula II: 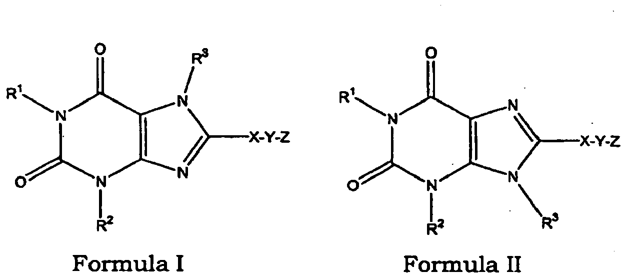 wherein:
R¹ and R² are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl, or optionally substituted alkynyl, with the proviso that when D is a covalent bond E cannot be alkoxy;
R³ is hydrogen, optionally substituted alkyl or optionally substituted cycloalkyl;
X is optionally substituted arylene or heteroarylene;
Y is a covalent bond or alkylene in which one carbon atom can be optionally replaced by -O-, -S-, or -NH-, and is optionally substituted by hydroxy, alkoxy, optionally substituted amino, or -COR, in which R is hydroxy, alkoxy or amino;
with the proviso that when the optional substitution is hydroxy or amino said substitution cannot be present on a carbon atom adjacent to a heteroatom; and
Z is hydrogen, optionally substituted monocyclic aryl or optionally substituted monocyclic heteroaryl;
with the proviso that
(a) Z is hydrogen only when Y is a covalent bond and X is optionally substituted 1,4-pyrazolene attached to the purine ring by a carbon atom; and
(b) when X is optionally substituted arylene, Z is an optionally substituted monocyclic heteroaryl other than optionally substituted imidazole,
for the preparation of a pharmaceutical composition for accelerating wound healing in a mammal.

2. The use of claim 1, wherein:
R¹ and R² are independently hydrogen, optionally substituted C₁₋₆ alkyl, or a group -D-E, in which D is a covalent bond or alkylene, and E is optionally substituted phenyl, optionally substituted cycloalkyl, optionally substituted alkenyl, or optionally substituted alkynyl, and
R3 is hydrogen.

3. The use of claim 2, wherein:
X is optionally substituted phenylene; and
Y is a covalent bond or C₁₋₆ alkylene in which one carbon atom can be optionally replaced by -O-, -S-, or -NH-.

4. The use of claim 3, wherein R¹ and R² are independently C₁₋₆ alkyl optionally substituted by cycloalkyl.

5. The use of claim 4, wherein R¹ and R² are n-propyl, Y is -OCH₂-, and Z is optionally substituted oxadiazole.

6. The use of claim 5, wherein
(i) Z is 5-(2-methoxyphenyl)-(1,2,4-oxadiazol-3-yl), namely 8-{4-[5-(2-methoxyphenyl)-[1,2,4]oxadiazol-3-ylmethoxy]phenyl}-1,3-dipropyl-1,3,7-trihydropurine-2,6-dione;
(ii) Z is 5-(3-methoxyphenyl)-(1,2,4-oxadiazol-3-yl), namely 8-{4-[5-(3-methoxyphenyl)-[1,2,4]oxadiazol-3-ylmethoxy]phenyl}-1,3-dipropyl-1,3,7-trihydropurine-2,6-dione; or
(iii) Z is 5-(4-fluorophenyl)-(1,2,4-oxadiazol-3-yl), namely 8-{4-[5-(4-fluorophenyl)-[1,2,4]oxadiazol-3-ylmethoxy]phenyl}-1,3-dipropyl-1,3,7-trihydropurine-2,6-dione.

7. The use of claim 2, wherein:
X is optionally substituted pyrazolene,
Y is a covalent bond, C₁₋₆ alkylene optionally substituted by hydroxy, alkoxy, optionally substituted amino, or -COR, in which R is hydroxy, alkoxy or amino; and
Z is hydrogen, optionally substituted phenyl, optionally substituted oxadiazolyl, optionally substituted isoxazolyl, or optionally substituted pyridyl.

8. The use of claim 7, wherein X is optionally substituted 1,4-pyrazolene.

9. The use of claim 8, wherein Z is optionally substituted phenyl or optionally substituted pyridyl.

10. The use of claim 9, wherein R¹ is C₁₋₆ alkyl optionally substituted by cycloalkyl, R² is hydrogen, and Y is -CH₂- or -CH(CH₃)-.

11. The use of claim 10, wherein
(i) R¹ is n-propyl, X is 1,4-pyrazolene, Y is -CH₂-, and Z is 3-trifluoromethylphenyl, namely 1-propyl-8-(1-{[3-(trifluoromethyl)-phenyl]methyl}pyrazol-4-yl)-1,3,7-trihydropurine-2,6-dione;
(ii) R¹ is n-propyl, X is 1,4-pyrazolene, Y is -CH₂-, and Z is phenyl, namely 1-propyl-8-[1-benzylpyrazol-4-yl]-1,3,7-trihydropurine-2,6-dione;
(iii) R¹ is n-butyl, X is 1,4-pyrazolene, Y is -CH₂-, and Z is 3-fluorophenyl, namely 1-butyl-8-(1-{[3-fluorophenyl]methyl}pyrazol-4-yl)-1,3,7-trihydropurine-2,6-dione;
(iv) R¹ is n-propyl, X is 1,4-pyrazolene, Y is -CH(CH₃)-, and Z is phenyl, namely 1-propyl-8-[1-(1-phenylethyl)pyrazol-4-yl]-1,3,7-trihydropurine-2,6-dione;
(v) R¹ is cyclopropylmethyl, X is 1,4-pyrazolene, Y is -CH₂-, and Z is 2-pyridyl, namely 1-(cyclopropylmethyl)-8-[1-(2-pyridylmethyl)pyrazol-4-yl]-1,3,7-trihydropurine-2,6-dione; or
(vi) R¹ is n-butyl, X is 1,4-pyrazolene, Y is -CH₂-, and Z is 6-trifluoromethylpyridin-3-yl, namely 1-n-butyl-8-[1-((6-trifluoromethyl)pyridin-3-ylmethyl)pyrazol-4-yl]-1,3,7-trihydropurine-2,6-dione.

12. The use of claim 9, wherein R¹ and R² are independently methyl, ethyl, n-propyl, or cyclopropylmethyl, and Y is methylene or ethylene which may be optionally substituted by hydroxy, alkoxy, optionally substituted amino, or -COR, in which R is hydroxy, alkoxy or amino.

13. The use of claim 12, wherein
(i) R¹ and R² are n-propyl, Y is -CH₂-, and Z is 3-(1,2,3,4-tetrazol-5-yl)phenyl, namely 1,3-dipropyl-8-{1-[(3-(1H-1,2,3,4-tetrazol-5-yl)phenyl)methyl]pyrazol-4-yl}-1,3,7-trihydropurine-2,6-dione;
(ii) R¹ is n-propyl, R² is ethyl, Y is -CH₂-, and Z is 3-trifluoromethylphenyl, namely 3-ethyl-1-propyl-8-{1-[(3-trifluoromethylphenyl)methyl]pyrazol-4-yl}-1,3,7-trihydropurine-2,6-dione;
(iii) R¹ and R² are n-propyl, Y is -CH(CH₃)-, and Z is 3-trifluoromethylphenyl, namely 1,3-dipropyl-8-(1-{1-[3-(trifluoromethyl)phenyl]ethyl}pyrazol-4-yl)-1,3,7-trihydropurine-2,6-dione;
(iv) R¹ and R² are n-propyl, Y is -CH₂-, and Z is 4-carboxyphenyl, namely 1,3-dipropyl-8-{1-[(4-carboxyphenyl)methyl]pyrazol-4-yl}-1,3,7-trihydropurine-2,6-dione;
(v) R¹ and R² are n-propyl, Y is -CH₂-, and Z is 3-carboxyphenyl, namely 3-{[4-(2,6-dioxo-1,3-dipropyl-1,3,7-trihydropurin-8-yl)-pyrazolyl]methyl}benzoic acid;
(vi) R¹ and R² are n-propyl, Y is -CH(CO₂H)-, and Z is phenyl, namely 2-[4-(2,6-dioxo-1,3-dipropyl-1,3,7-trihydropurin-8-yl)pyrazolyl]-2-phenylacetic acid;
(vii) R¹ is cyclopropylmethyl, R² is methyl, Y is -CH₂-, and Z is 3-trifluoromethylphenyl, 1-cyclopropylmethyl-3-methyl-8-{1-[(3-trifluoromethylphenyl)methyl]pyrazol-4-yl}-1,3,7-trihydropurine-2,6-dione;
(viii) R¹ and R² are methyl, Y is -CH₂-, and Z is 3-fluorophenyl, namely 1,3-dimethyl-8-{1-[(3-fluorophenyl)methyl]pyrazol-4-yl}-1,3,7-trihydropurine-2,6-dione;
(ix) R¹ is n-propyl, R² is methyl, Y is -CH₂-, and Z is 3-trifluoromethylphenyl, namely 3-methyl-1-propyl-8-{1-[(3-trifluoromethylphenyl)methyl]pyrazol-4-yl}-1,3,7-trihydropurine-2,6-dione;
(x) R¹ and R² are n-propyl, Y is -CH₂-, and Z is 3-(trifluoromethyl)phenyl, namely 1,3-dipropyl-8-(1-{[3-(trifluoromethyl)phenyl]methyl}pyrazol-4-yl)-1,3,7-trihydropurine-2,6-dione;
(xi) R¹ and R² are n-propyl, Y is -CH₂-, and Z is 3-fluorophenyl, namely 1,3-dipropyl-8-{1-[(3-fluorophenyl)methyl]pyrazol-4-yl}-1,3,7-trihydropurine-2,6-dione;
(xii) R¹ is ethyl, R² is methyl, Y is -CH₂-, and Z is 3-fluorophenyl, namely 1-ethyl-3-methyl-8-{1-[(3-fluorophenyl)methyl]pyrazol-4-yl}-1,3,7-trihydropurine-2,6-dione; or
(xiii) R¹ and R² are n-propyl, Y is -CH₂-, and Z is 2-methoxyphenyl, 1,3-dipropyl-8-{1-[(2-methoxyphenyl)methyl]pyrazol-4-yl}-1,3,7-trihydropurine-2,6-dione.

14. The use of claim 8, wherein Z is optionally substituted oxadiazole.

15. The use of claim 14, wherein R¹ is C₁₋₆ alkyl optionally substituted by cycloalkyl, R² is H, and Y is -CH₂- or -CH(CH₃)-.

16. The use of claim 15, wherein
(i) R¹ is n-propyl, X is 1,4-pyrazolene, Y is -CH₂-, and Z is 5-(4-chlorophenyl)-[1,2,4]-oxadiazol-3-yl, namely 8-(1-{[5-(4-chlorophenyl)(1,2,4-oxadiazol-3-yl)]methyl}pyrazol-4-yl)-1-propyl-1,3,7-trihydropurine-2,6-dione; or
(ii) R¹ is n-butyl, X is 1,4-pyrazolene, Y is -CH₂-, and Z is 5-(4-chlorophenyl)-[1,2,4]-oxadiazol-3-yl, namely 8-(1-{[5-(4-chlorophenyl)(1,2,4-oxadiazol-3-yl)]methyl}pyrazol-4-yl)-1-butyl-1,3,7-trihydropurine-2,6-dione.

17. The use of claim 14, wherein R¹ and R² are independently C₁₋₆ alkyl optionally substituted by cycloalkyl and Y is -CH₂- or -CH(CH₃)-.

18. The use of claim 17, wherein
(i) R¹ and R² are n-propyl, Y is -CH₂-, and Z is 3-(4-chlorophenyl)-[1,2,4]-oxadiazol-5-yl, namely 8-(1-{[3-(4-chlorophenyl)(1,2,4-oxadiazol-5-yl)]methyl)pyrazol-4-yl]-1,3-dipropyl-1,3,7-trihydropurine-2,6-dione; or
(ii) R¹ is n-propyl, R² is ethyl, Y is -CH₂-, and Z is 3-(4-chlorophenyl)-[1,2,4]-oxadiazol-5-yl, namely 8-(1-{[3-(4-chlorophenyl)(1,2,4-oxadiazol-5-yl)]methyl}pyrazol-4-yl)-3-ethyl-1-propyl-1,3,7-trihydropurine-2,6-dione.

19. The use of claim 8, wherein Z is hydrogen.

20. The use of claim 19, wherein R¹ and R² are independently C₁₋₆ alkyl optionally substituted by cycloalkyl, and Y is a covalent bond.

21. The use of claim 20, wherein
(i) R¹ and R² are n-propyl, namely 1,3-dipropyl-8-pyrazol-4-yl-1,3,7-trihydropurine-2,6-dione; or
(ii) R² is sec-butyl, and R¹ is methyl, namely 1-methyl-3-sec-butyl-8-pyrazol-4-yl-1,3,7-trihydropurine-2,6-dione.

22. The use of claim 8, wherein Z is optionally substituted isoxazolyl.

23. The use of claim 22, wherein R¹ and R² are independently C₁₋₆ alkyl optionally substituted by cycloalkyl, and Y is -CH₂-, -CH(CH₃)-, or a covalent bond.

24. The use of claim 23, wherein
(i) R¹ and R² are n-propyl, Y is -CH₂-, and Z is 5-(4-trifluoromethylphenyl)isoxazol-3-yl, namely 1,3-dipropyl-8-[1-({5-[4-(trifluoromethyl)phenyl]isoxazol-3-yl}methyl)pyrazol-4-yl]-1,3,7-trihydropurine-2,6-dione; or
(ii) R¹ is n-propyl, R² is ethyl, Y is -CH₂-, and Z is 5-(4-chlorophenyl)-isoxazol-3-yl, namely 8-(1-{[5-(4-chlorophenyl)isoxazol-3-yl]methyl}pyrazol-4-yl)-3-ethyl-1-propyl-1,3,7-trihydropurine-2,6-dione.

25. The use of claim 8, wherein Z is optionally substituted pyridyl.

26. The use of claim 25, wherein
R¹ and R² are independently C₁₋₆ alkyl optionally substituted by cycloalkyl, and Y is -CH₂-, -CH(CH₃)-, or a covalent bond.

27. The use of claim 26, wherein
(i) R¹ and R² are n-propyl, Y is -CH₂-, and Z is pyrid-2-yl, namely 1,3-dipropyl-8-[1-(2-pyridylmethyl)pyrazol-4-yl]-1,3,7-trihydropurine-2,6-dione;
(ii) R¹ and R² are n-propyl, Y is -CH₂-, and Z is 6-triffuoromethylpyrid-3-yl, namely 1,3-dipropyl-8-(1-{[6-(trifluoromethyl)(3-pyridyl)]-methyl}pyrazol-4-yl)-1,3,7-trihydropurine-2,6-dione;
(iii) R¹ and R² are n-propyl, Y is -CH₂-, and Z is 6-carboxy-pyrid-2-yl, namely 6-{[4-(2,6-dioxo-1,3-dipropyl-1,3,7-trihydropurin-8-yl)pyrazolyl]methyl}pyridine-2-carboxylic acid;
(iv) R¹ is n-propyl, R² is ethyl, Y is -CH₂-, and Z is 2-pyridyl, namely 3-ethyl-1-propyl-8-[1-(2-pyridylmethyl)pyrazol-4-yl]-1,3,7-trihydropurine-2,6-dione;
(v) R¹ is n-propyl, R² is ethyl, Y is -CH₂-, and Z is 6-(trifluoromethyl)-pyrid-3-yl, namely 3-ethyl-1-propyl-8-(1-{[6-(trifluoromethyl)(3-pyridyl)]methyl}pyrazol-4-yl)-1,3,6-trihydropurine-2,6-dione;
(vi) R¹ is cyclopropylmethyl, R² is ethyl, Y is -CH₂-, and Z is 6-(trifluoromethyl)-pyrid-3-yl, namely 1-(cyclopropylmethyl)-3-ethyl-8-(1-{[6-(trifluoromethyl)(3-pyridyl)]methyl}pyrazol-4-yl)-1,3,7-trihydropurine-2,6-dione; or
(vii) R¹ is 2-methylpropyl, R² is ethyl, Y is -CH₂-, and Z is 6-(trifluoromethyl)-pyrid-3-yl, namely 3-ethyl-1-(2-methylpropyl)-8-(1-{[6-(trifluoromethyl) (3-pyridyl)]methyl}pyrazol-4-yl)-1,3,7-trihydropurine-2,6-dione.

28. The use of claim 1, wherein the mammal is human, or is a domesticated animal.

29. The use of claim 1, wherein the pharmaceutical composition is for topical administration, systemic administration, or administration directly to the wound.

30. The use of claim 1, wherein the wound is caused by mechanical, chemical or thermal trauma.

31. The use of claim 30, wherein the wound is the result of a surgical incision.

32. The use of claim 30, wherein the wound is selected from the group consisting of contusions, burns, incisions, and lacerations.

33. The use of claim 1, wherein the wound is associated with a disease or disorder.

34. The use of claim 33, wherein the wound is a diabetic ulcer.

35. A pharmaceutical composition for use in augmenting wound healing and suitable for topical delivery comprising a therapeutically effective amount of an A_{2B} receptor antagonist having the structure of Formula I or Formula II: 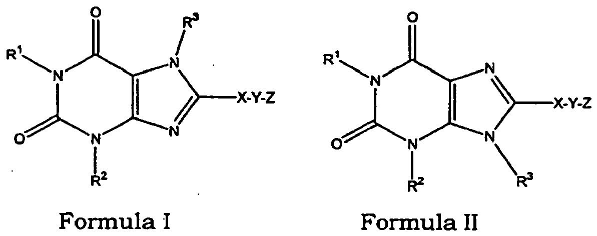 wherein:
R¹ and R² are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl, or optionally substituted alkynyl, with the proviso that when D is a covalent bond E cannot be alkoxy;
R³ is hydrogen, optionally substituted alkyl or optionally substituted cycloalkyl;
X is optionally substituted arylene or heteroarylene;
Y is a covalent bond or alkylene in which one carbon atom can be optionally replaced by -O-, -S-, or -NH-, and is optionally substituted by hydroxy, alkoxy, optionally substituted amino, or -COR, in which R is hydroxy, alkoxy or amino;
with the proviso that when the optional substitution is hydroxy or amino said substitution cannot be present on a carbon atom adjacent to a heteroatom; and
Z is hydrogen, optionally substituted monocyclic aryl or optionally substituted monocyclic heteroaryl;
with the proviso that
(a) Z is hydrogen only when Y is a covalent bond and X is optionally substituted 1,4-pyrazolene attached to the purine ring by a carbon atom; and
(b) when X is optionally substituted arylene, Z is an optionally substituted monocyclic heteroaryl other than optionally substituted imidazole, and
a pharmaceutically acceptable carrier.

36. The pharmaceutical composition for use in augmenting wound healing and suitable for topical delivery according to claim 35 which is an ointment, cream or gel.

## Patentansprüche

1. Verwendung eines A₂B-Rezeptor-Antagonisten mit der Struktur von Formel I oder Formel II: 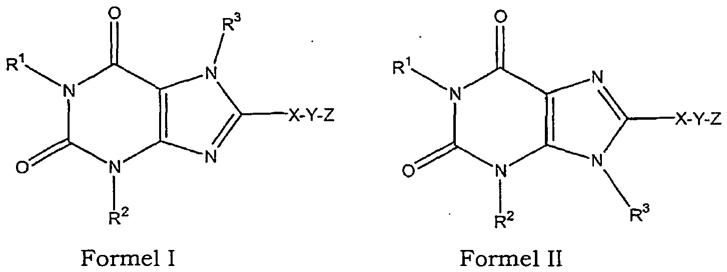 worin:
R¹ und R² unabhängig voneinander ausgewählt sind aus Wasserstoffatom, gegebenenfalls substituierter Alkylgruppe oder einer Gruppe -D-E, worin D eine kovalente Bindung oder eine Alkylengruppe ist und E eine gegebenenfalls substituierte Alkoxygruppe, gegebenenfalls substituierte Cycloalkylgruppe, gegebenenfalls substituierte Arylgruppe, gegebenenfalls substituierte Heteroarylgruppe, gegebenenfalls substituierte Heterocyclylgruppe, gegebenenfalls substituierte Alkenylgruppe oder gegebenenfalls substituierte Alkinylgruppe ist, mit der Maßgabe, dass, wenn D eine kovalente Bindung ist, E keine Alkoxygruppe sein kann,
R³ ein Wasserstoffatom, eine gegebenenfalls substituierte Alkylgruppe oder gegebenenfalls substituierte Cycloalkylgruppe ist,
X eine gegebenenfalls substituierte Arylengruppe oder Heteroarylengruppe ist,
Y eine kovalente Bindung oder eine Alkylengruppe ist, worin ein Kohlenstoffatom gegebenenfalls durch -O-, -S- oder -NH- ersetzt sein kann, und gegebenenfalls durch Hydroxygruppe,
Alkoxygruppe, gegebenenfalls substituierte Aminogruppe oder -COR-Gruppe substituiert ist, worin R eine Hydroxygruppe, Alkoxygruppe oder Aminogruppe ist,
mit der Maßgabe, dass, wenn die gegebenenfalls vorhandene Substitution eine Hydroxygruppe oder Aminogruppe ist, die Substitution nicht an einem Kohlenstoffatom vorliegen kann, das zu einem Heteroatom benachbart ist, und
Z ein Wasserstoffatom, eine gegebenenfalls substituierte monocyclische Arylgruppe oder gegebenenfalls substituierte monocyclische Heteroarylgruppe ist,
mit der Maßgabe, dass
(a) Z nur dann ein Wasserstoffatom ist, wenn Y eine kovalente Bindung ist und X eine gegebenenfalls substituierte 1,4-Pyrazolengruppe ist, die an den Purinring durch ein Kohlenstoffatom gebunden ist, und
(b) wenn X eine gegebenenfalls substituierte Arylengruppe ist, Z eine gegebenenfalls substituierte monocyclische Heteroarylgruppe ist, die von einer gegebenenfalls substituierten Imidazolgruppe verschieden ist,
zur Herstellung einer pharmazeutischen Zusammensetzung zur Beschleunigung von Wundheilung bei einem Säuger.

2. Verwendung nach Anspruch 1, worin:
R¹ und R² unabhängig voneinander ein Wasserstoffatom, eine gegebenenfalls substituierte C₁₋₆-Alkylgruppe oder eine Gruppe -D-E sind, worin D eine kovalente Bindung oder eine Alkylengruppe ist und E eine gegebenenfalls substituierte Phenylgruppe, gegebenenfalls substituierte Cycloalkylgruppe, gegebenenfalls substituierte Alkenylgruppe oder gegebenenfalls substituierte Alkinylgruppe ist, und
R³ ein Wasserstoffatom ist.

3. Verwendung nach Anspruch 2, worin:
X eine gegebenenfalls substituierte Phenylengruppe ist und
Y eine kovalente Bindung oder eine C₁₋₆-Alkylengruppe ist, worin ein Kohlenstoffatom gegebenenfalls durch -O-, -S- oder -NH- ersetzt sein kann.

4. Verwendung nach Anspruch 3, worin R¹ und R² unabhängig voneinander eine C₁₋₆-Alkylgruppe sind, die gegebenenfalls durch eine Cycloalkylgruppe substituiert ist.

5. Verwendung nach Anspruch 4, worin R¹ und R² eine n-Propylgruppe sind, Y eine -OCH₂--Gruppe ist und Z eine gegebenenfalls substituierte Oxadiazolgruppe ist.

6. Verwendung nach Anspruch 5, worin
(i) Z eine 5-(2-Methoxyphenyl)-(1,2,4-oxadiazol-3-yl)-Gruppe ist, namentlich 8-{4-[5-(2-Methoxyphenyl)-[1,2,4]oxadiazol-3-ylmethoxy]phenyl}-1,3-dipropyl-1,3,7-trihydropurin-2,6-dion,
(ii) Z eine 5-(3-Methoxyphenyl)-(1,2,4-oxadiazol-3-yl)-Gruppe ist, namentlich 8-{4-[3-(3-Methoxyphenyl)-[1,2,4]oxadiazol-3-ylmethoxy]phenyl}-1,3-dipropyl-1,3,7-trihydropurin-2,6-dion, oder
(iii) Z eine 5-(4-Fluorphenyl)-(1,2,4-oxadiazol-3-yl)-Gruppe ist, namentlich 8-{4-[5-(4-Fluorphenyl)-[1,2,4]oxadiazol-3-ylmethoxy]phenyl}-1,3-dipropyl-1,3,7-trihydropurin-2,6-dion.

7. Verwendung nach Anspruch 2, worin:
X eine gegebenenfalls substituierte Pyrazolengruppe ist,
Y eine kovalente Bindung oder eine C₁₋₆-Alkylengruppe ist, die gegebenenfalls durch Hydroxygruppe, Alkoxygruppe, gegebenenfalls substituierte Aminogruppe oder -COR-Gruppe substituiert ist, worin R eine Hydroxygruppe, Alkoxygruppe oder Aminogruppe ist, und
Z ein Wasserstoffatom, eine gegebenenfalls substituierte Phenylgruppe, gegebenenfalls substituierte Oxadiazolylgruppe, gegebenenfalls substituierte Isoxazolylgruppe oder gegebenenfalls substituierte Pyridylgruppe ist.

8. Verwendung nach Anspruch 7, worin X eine gegebenenfalls substituierte 1,4-Pyrazolengruppe ist.

9. Verwendung nach Anspruch 8, worin Z eine gegebenenfalls substituierte Phenylgruppe oder gegebenenfalls substituierte Pyridylgruppe ist.

10. Verwendung nach Anspruch 9, worin R¹ eine C₁₋₆-Alkylgruppe ist, die gegebenenfalls durch eine Cycloalkylgruppe substituiert ist, R² ein Wasserstoffatom ist und Y eine -CH₂-- oder -CH(CH₃)--Gruppe ist.

11. Verwendung nach Anspruch 10, worin
(i) R¹ eine n-Propylgruppe ist, X eine 1,4-Pyrazolengruppe ist, Y eine -CH₂--Gruppe ist und Z eine 3-Trifluormethylphenylgruppe ist, namentlich 1-Propyl-8-(1-{[3-(trifluormethyl)-phenyl]-methyl}pyrazol-4-yl)-1,3,7-trihydropurin-2, 6-dion,
(ii) R¹ eine n-Propylgruppe ist, X eine 1,4-Pyrazolengruppe ist, Y eine -CH₂--Gruppe ist und Z eine Phenylgruppe ist, namentlich 1-Propyl-8-[1-benzylpyrazol-4-yl]-1,3,7-trihydropurin-2,6-dion,
(iii) R¹ eine n-Butylgruppe ist, X eine 1,4-Pyrazolengruppe ist, Y eine -CH₂--Gruppe ist und Z eine 3-Fluorphenylgruppe ist, namentlich 1-Butyl-8-(1-{[3-fluorphenyl]methyl}pyrazol-4-yl)-1,3,7-trihydropurin-2,6-dion,
(iv) R¹ eine n-Propylgruppe ist, X eine 1,4-Pyrazolengruppe ist, Y eine -CH(CH₃)--Gruppe ist und Z eine Phenylgruppe ist, namentlich 1-Propyl-8-[1-(1-phenylethyl)pyrazol-4-yl]-1,3,7-trihydropurin-2,6-dion,
(v) R¹ eine Cyclopropylmethylgruppe ist, X eine 1,4-Pyrazolengruppe ist, Y eine -CH₂--Gruppe ist und Z eine 2-Pyridylgruppe ist, namentlich 1-(Cyclopropylmethyl)-8-[1-(2-pyridylmethyl)pyrazol-4-yl]-1, 3, 7-trihydropurin-2, 6-dion, oder
(vi) R¹ eine n-Butylgruppe ist, X eine 1,4-Pyrazolengruppe ist, Y eine -CH₂--Gruppe ist und Z eine 6-Trifluormethylpyridin-3-ylgruppe ist, namentlich 1-n-Butyl-8-[1-((6-trifluormethyl)pyridin-3-ylmethyl)pyrazol-4-yl]-1,3,7-trihydropurin-2,6-dion.

12. Verwendung nach Anspruch 9, worin R¹ und R² unabhängig voneinander eine Methylgruppe, Ethylgruppe, n-Propylgruppe oder Cyclopropylmethylgruppe sind und Y eine Methylengruppe oder Ethylengruppe ist, die gegebenenfalls durch eine Hydroxygruppe, Alkoxygruppe, gegebenenfalls substituierte Aminogruppe oder -COR-Gruppe substituiert sein kann, worin R eine Hydroxygruppe, Alkoxygruppe oder Aminogruppe ist.

13. Verwendung nach Anspruch 12, worin
(i) R¹ und R² n-Propylgruppen sind, Y eine -CH₂--Gruppe ist und Z eine 3-(1,2,3,4-Tetrazol-5-yl)phenylgruppe ist, namentlich 1,3-Dipropyl-8-{1-[(3-(1H-1,2,3,4-tetrazol-5-yl)phenyl)methyl]pyrazol-4-yl}-1,3,7-trihydropurin-2,6-dion,
(ii) R¹ eine n-Propylgruppe ist, R² eine Ethylgruppe ist, Y eine -CH₂--Gruppe ist und Z eine 3-Trifluormethylphenylgruppe ist, namentlich 3-Ethyl-1-propyl-8-{1-[(3-trifluormethylphenyl) methyl] pyrazol-4-yl}-1,3,7-trihydropurin-2,6-dion,
(iii) R¹ und R² n-Propylgruppen sind, Y eine -CH(CH₃)--Gruppe ist und Z eine 3-Trifluormethylphenylgruppe ist, namentlich 1,3-Dipropyl-8-(1-{1-[3-(trifluormethyl) phenyl] ethyl}pyrazol-4-yl)-1,3,7-trihydropurin-2,6-dion,
(iv) R¹ und R² n-Propylgruppen sind, Y eine -CH₂--Gruppe ist und Z eine 4-Carboxyphenylgruppe ist, namentlich 1,3-Dipropyl-8-{1-[(4-carboxyphenyl)methyl]pyrazol-4-yl}-1,3,7-trihydropurin-2,6-dion,
(v) R¹ und R² n-Propylgruppen sind, Y eine -CH₂--Gruppe ist und Z eine 3-Carboxyphenylgruppe ist, namentlich 3-{[4-(2,6-Dioxo-1,3-dipropyl-1, 3, 7-trihydropurin-8-yl)-pyrazolyl]-methyl}benzoesäure,
(vi) R¹ und R² n-Propylgruppen sind, Y eine -CH(CO₂H)--Gruppe ist und Z eine Phenylgruppe ist, namentlich 2-[4-(2,6-Dioxo-1,3-dipropyl-1,3,7-trihydropurin-8-yl) pyrazolyl]-2-phenylessigsäure,
(vii) R¹ eine Cyclopropylmethylgruppe ist, R² eine Methylgruppe ist, Y eine -CH₂--Gruppe ist und Z eine 3-Trifluormethylphenylgruppe ist, namentlich 1-Cyclopropylmethyl-3-methyl-8-{1-[(3-trifluormethylphenyl)-methyl]pyrazol-4-yl}-1,3,7-trihydropurin-2,6-dion,
(viii) R¹ und R² Methylgruppen sind, Y eine -CH₂--Gruppe ist und Z eine 3-Fluorphenylgruppe ist, namentlich 1,3-Dimethyl-8-{1-[(3-fluorphenyl) methyl] pyrazol-4-yl}-1,3,7-trihydropurin-2,6-dion,
(ix) R¹ eine n-Propylgruppe ist, R² eine Methylgruppe ist, Y eine -CH₂--Gruppe ist und Z eine 3-Trifluormethylphenylgruppe ist, namentlich 3-Methyl-1-propyl-8-{1-[(3-trifluormethylphenyl) methyl] pyrazol-4-yl}-1, 3 , 7-trihydropurin-2, 6-dion,
(x) R¹ und R² n-Propylgruppen sind, Y eine -CH₂--Gruppe ist und Z eine 3-(Trifluormethyl)phenylgruppe ist, namentlich 1,3-Dipropyl-8-(1-{[3-(trifluormethyl)phenyl]methyl}pyrazol-4-yl)-1,3,7-trihydropurin-2,6-dion,
(xi) R¹ und R² n-Propylgruppen sind, Y eine -CH₂--Gruppe ist und Z eine 3-Fluorphenylgruppe ist, namentlich 1,3-Dipropyl-8-(1-[(3-fluorphenyl)methyl]pyrazol-4-yl)-1,3,7-trihydropurin-2, 6-dion,
(xii) R¹ eine Ethylgruppe ist, R² eine Methylgruppe ist, Y eine -CH₂--Gruppe ist und Z eine 3-Fluorphenylgruppe ist, namentlich 1-Ethyl-3-methyl-8-{1-[(3-fluorphenyl)methyl]-pyrazol-4-yl}-1,3,7-trihydropurin-2,6-dion, oder
(xiii) R¹ und R² n-Propylgruppen sind, Y eine -CH₂--Gruppe ist und Z eine 2-Methoxyphenylgruppe ist, namentlich 1,3-Dipropyl-8-{1-[(2-methoxyphenyl)methyl]pyrazol-4-yl}-1,3, 7-trihydropurin-2,6-dion.

14. Verwendung nach Anspruch 8, worin Z eine gegebenenfalls substituierte Oxadiazolgruppe ist.

15. Verwendung nach Anspruch 14, worin R¹ eine C₁₋₆-Alkylgruppe ist, die gegebenenfalls durch eine Cycloalkylgruppe substituiert ist, R² ein H-Atom ist und Y eine -CH₂-- oder -CH(CH₃)--Gruppe ist.

16. Verwendung nach Anspruch 15, worin
(i) R¹ eine n-Propylgruppe ist, X eine 1,4-Pyrazolengruppe ist, Y eine -CH₂--Gruppe ist und Z eine 5-(4-Chlorphenyl)-[1,2,4]-oxadiazol-3-ylgruppe ist, namentlich 8-(1-{[5-(4-Chlorphenyl)(1,2,4-oxadiazol-3-yl)]methyl}pyrazol-4-yl)-1-propyl-1,3,7-trihydropurin-2,6-dion, oder
(ii) R¹ eine n-Butylgruppe ist, X eine 1,4-Pyrazolengruppe ist, Y eine -CH₂--Gruppe ist und Z eine 5-(4-Chlorphenyl)-[1,2,4]-oxadiazol-3-ylgruppe ist, namentlich 8-(1-{[5-(4-Chlorphenyl)(1,2,4-oxadiazol-3-yl)]methyl}pyrazol-4-yl)-1-butyl-1,3,7-trihydropurin-2,6-dion.

17. Verwendung nach Anspruch 14, worin R¹ und R² unabhängig voneinander eine C₁₋₆-Alkylgruppe sind, die gegebenenfalls durch eine Cycloalkylgruppe substituiert ist, und Y eine -CH₂-- oder -CH(CH₃)--Gruppe ist.

18. Verwendung nach Anspruch 17, worin
(i) R¹ und R² n-Propylgruppen sind, Y eine -CH₂--Gruppe ist und Z eine 3-(4-Chlorphenyl)-[1,2,4]-oxadiazol-5-ylgruppe ist, namentlich 8-(1-{[3-(4-Chlorphenyl)(1,2,4-oxadiazol-5-yl)]methyl}pyrazol-4-yl)-1,3-dipropyl-1,3, 7-trihydropurin-2, 6-dion, oder
(ii) R¹ eine n-Propylgruppe ist, R² eine Ethylgruppe ist, Y eine -CH₂--Gruppe ist und Z eine 3-(4-Chlorphenyl)-[1,2,4]-oxadiazol-5-ylgruppe ist, namentlich 8-(1-{[3-(4-chlorphenyl)(1,2,4-oxadiazol-5-yl)]methyl}pyrazol-4-yl)-3-ethyl-1-propyl-1,3,7-trihydropurin-2, 6-dion.

19. Verwendung nach Anspruch 8, worin Z ein Wasserstoffatom ist.

20. Verwendung nach Anspruch 19, worin R¹ und R² unabhängig voneinander eine C₁₋₆-Alkylgruppe sind, die gegebenenfalls durch eine Cycloalkylgruppe substituiert ist, und Y eine kovalente Bindung ist.

21. Verwendung nach Anspruch 20, worin
(i) R¹ und R² n-Propylgruppen sind, namentlich 1,3-Dipropyl-8-pyrazol-4-yl-1,3,7-trihydropurin-2,6-dion, oder
(ii) R² eine sec-Butylgruppe ist und R¹ eine Methylgruppe ist, namentlich 1-Methyl-3-sec-butyl-8-pyrazol-4-yl-1,3,7-trihydropurin-2,6-dion.

22. Verwendung nach Anspruch 8, worin Z eine gegebenenfalls substituierte Isoxazolylgruppe ist.

23. Verwendung nach Anspruch 22, worin R¹ und R² unabhängig voneinander eine C₁₋₆-Alkylgruppe sind, die gegebenenfalls durch eine Cycloalkylgruppe substituiert ist, und Y eine -CH₂--Gruppe, -CH(CH₃)--Gruppe oder eine kovalente Bindung ist.

24. Verwendung nach Anspruch 23, worin
(i) R¹ und R² n-Propylgruppen sind, Y eine -CH₂--Gruppe ist und Z eine 5-(4-Trifluormethylphenyl)isoxazol-3-ylgruppe ist, namentlich 1,3-Dipropyl-8-[1-({5-[4-(trifluormethyl)phenyl]-isoxazol-3-yl}methyl) pyrazol-4-yl]-1,3,7-trihydropurin-2,6-dion, oder
(ii) R¹ eine n-Propylgruppe ist, R² eine Ethylgruppe ist, Y eine -CH₂--Gruppe ist und Z eine 5-(4-Chlorphenyl)-isoxazol-3-ylgruppe ist, namentlich 8-(1-{[5-(4-Chlorphenyl)isoxazol-3-yl]methyl}-pyrazol-4-yl)-3-ethyl-1-propyl-1,3,7-trihydropurin-2,6-dion.

25. Verwendung nach Anspruch 8, worin Z eine gegebenenfalls substituierte Pyridylgruppe ist.

26. Verwendung nach Anspruch 25, worin
R¹ und R² unabhängig voneinander eine C₁-₆-Alkylgruppe sind, die gegebenenfalls durch eine Cycloalkylgruppe substituiert ist, und Y eine -CH₂--Gruppe, eine -CH(CH₃)--Gruppe oder eine kovalente Bindung ist.

27. Verwendung nach Anspruch 26, worin
(i) R¹ und R² n-Propylgruppen sind, Y eine -CH₂--Gruppe ist und Z eine Pyrid-2-ylgruppe ist, namentlich 1,3-Dipropyl-8-[1-(2-pyridylmethyl)pyrazol-4-yl]-1,3,7-trihydropurin-2,6-dion,
(ii) R¹ und R² n-Propylgruppen sind, Y eine -CH₂--Gruppe ist und Z eine 6-Trifluormethylpyrid-3-ylgruppe ist, namentlich 1,3-Dipropyl-8-(1-{[6-(trifluormethyl) (3-pyridyl)]-methyl}pyrazol-4-yl)-1, 3, 7-trihydropurin-2, 6-dion,
(iii) R¹ und R² n-Propylgruppen sind, Y eine -CH₂--Gruppe ist und Z eine 6-Carboxy-pyrid-2-ylgruppe ist, namentlich 6-{[4-(2,6-Dioxo-1,3-dipropyl-1,3,7-trihydropurin-8-yl)pyrazolyl]-methyl}pyridin-2-carbonsäure,
(iv) R¹ eine n-Propylgruppe ist, R² eine Ethylgruppe ist, Y eine -CH₂--Gruppe ist, und Z eine 2-Pyridylgruppe ist, namentlich 3-Ethyl-1-propyl-8-[1-(2-pyridylmethyl)pyrazol-4-yl]-1,3,7-trihydropurin-2,6-dion,
(v) R¹ eine n-Propylgruppe ist, R² eine Ethylgruppe ist, Y eine -CH₂--Gruppe ist und Z eine 6-(Trifluormethyl)-pyrid-3-ylgruppe ist, namentlich 3-Ethyl-1-propyl-8-(1-{[6-(trifluormethyl) (3-pyridyl)]methyl}pyrazol-4-yl)-1,3,7-trihydropurin-2, 6-dion,
(vi) R¹ eine Cyclopropylmethylgruppe ist, R² eine Ethylgruppe ist, Y eine -CH₂--Gruppe ist und Z eine 6-(Trifluormethyl)-pyrid-3-ylgruppe ist, namentlich 1-(Cyclopropylmethyl)-3-ethyl-8-(1-{[6-(trifluormethyl) (3-pyridyl)]methyl}pyrazol-4-yl)-1,3,7-trihydropurin-2,6-dion, oder
(vii) R¹ eine 2-Methylpropylgruppe ist, R² eine Ethylgruppe ist, Y eine -CH₂--Gruppe ist und Z eine 6-(Trifluormethyl)-pyrid-3-ylgruppe ist, namentlich 3-Ethyl-1-(2-methylpropyl)-8-(1-{[6-(trifluormethyl) (3-pyridyl)] methyl}pyrazol-4-yl)-1,3,7-trihydropurin-2,6-dion.

28. Verwendung nach Anspruch 1, worin der Säuger ein Mensch oder ein domestiziertes Tier ist.

29. Verwendung nach Anspruch 1, worin die pharmazeutische Zusammensetzung einer topischen Verabreichung, einer systemischen Verabreichung oder einer Verabreichung direkt an die Wunde dient.

30. Verwendung nach Anspruch 1, worin die Wunde durch eine mechanische, chemische oder thermische Verletzung verursacht wird.

31. Verwendung nach Anspruch 30, worin die Wunde das Ergebnis eines operativen Schnitts ist.

32. Verwendung nach Anspruch 30, worin die Wunde ausgewählt ist aus der Gruppe, bestehend aus Quetschungen, Verbrennungen, Schnitten und Fleischwunden.

33. Verwendung nach Anspruch 1, worin die Wunde mit einer Krankheit oder Erkrankung assoziiert ist.

34. Verwendung nach Anspruch 33, worin die Wunde ein diabetisches Geschwür ist.

35. Pharmazeutische Zusammensetzung für eine Verwendung zur Steigerung von Wundheilung, die für eine topische Verabreichung geeignet ist und eine therapeutisch wirksame Menge eines A_{2B}-Rezeptor-Antagonisten mit der Struktur von Formel I oder Formel II: 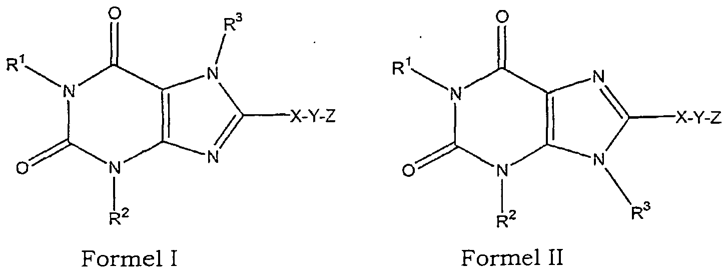 worin:
R¹ und R² unabhängig voneinander ausgewählt sind aus Wasserstoffatom, gegebenenfalls substituierter Alkylgruppe oder einer Gruppe -D-E, worin D eine kovalente Bindung oder eine Alkylengruppe ist und E eine gegebenenfalls substituierte Alkoxygruppe, gegebenenfalls substituierte Cycloalkylgruppe, gegebenenfalls substituierte Arylgruppe, gegebenenfalls substituierte Heteroarylgruppe, gegebenenfalls substituierte Heterocyclylgruppe, gegebenenfalls substituierte Alkenylgruppe oder gegebenenfalls substituierte Alkinylgruppe ist, mit der Maßgabe, dass, wenn D eine kovalente Bindung ist, E keine Alkoxygruppe sein kann,
R³ ein Wasserstoffatom, eine gegebenenfalls substituierte Alkylgruppe oder gegebenenfalls substituierte Cycloalkylgruppe ist,
X eine gegebenenfalls substituierte Arylengruppe oder Heteroarylengruppe ist,
Y eine kovalente Bindung oder eine Alkylengruppe ist, worin ein Kohlenstoffatom gegebenenfalls durch -O-, -S- oder -NH- ersetzt sein kann, und gegebenenfalls durch Hydroxygruppe, Alkoxygruppe, gegebenenfalls substituierte Aminogruppe oder -COR-Gruppe substituiert ist, worin R eine Hydroxygruppe, Alkoxygruppe oder Aminogruppe ist,
mit der Maßgabe, dass, wenn die gegebenenfalls vorhandene Substitution eine Hydroxygruppe oder Aminogruppe ist, die Substitution nicht an einem Kohlenstoffatom vorliegen kann, das zu einem Heteroatom benachbart ist, und
Z ein Wasserstoffatom, eine gegebenenfalls substituierte monocyclische Arylgruppe oder gegebenenfalls substituierte monocyclische Heteroarylgruppe ist,
mit der Maßgabe, dass
(a) Z nur dann ein Wasserstoffatom ist, wenn Y eine kovalente Bindung ist und X eine gegebenenfalls substituierte 1,4-Pyrazolengruppe ist, die an den Purinring durch ein Kohlenstoffatom gebunden ist, und
(b) wenn X eine gegebenenfalls substituierte Arylengruppe ist, Z eine gegebenenfalls substituierte monocyclische Heteroarylgruppe ist, die von einer gegebenenfalls substituierten Imidazolgruppe verschieden ist,
und einen pharmazeutisch verträglichen Träger umfasst.

36. Pharmazeutische Zusammensetzung für eine Verwendung zur Steigerung von Wundheilung, die für eine topische Verabreichung geeignet ist, nach Anspruch 35, die als Salbe, Creme oder Gel vorliegt.

## Revendications

1. Utilisation d'un antagoniste de récepteur A_{2B} ayant la structure selon Formule I ou Formule II: 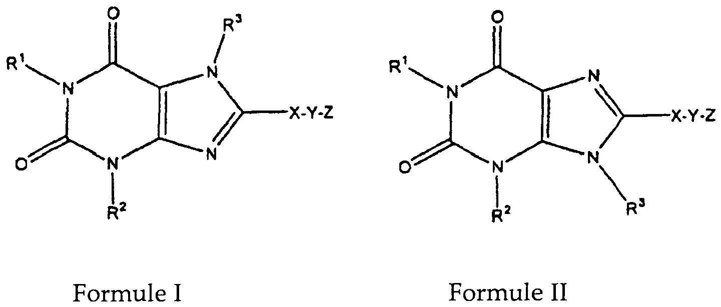 où:
R¹ et R² sont choisis indépendamment parmi hydrogène, alkyle éventuellement substitué, ou un groupe -D-E, dans lequel D est une liaison covalente ou un alkylène, et E est un alcoxy éventuellement substitué, un cycloalkyle éventuellement substitué, un aryle éventuellement substitué, un hétéroaryle éventuellement substitué, un hétérocyclyle éventuellement substitué, un alcényle éventuellement substitué, ou un alcynyle éventuellement substitué, avec pour condition que quand D est une liaison covalente E ne peut pas alcoxy;
R³ est hydrogène, alkyle éventuellement substitué ou cycloalkyle éventuellement substitué;
X est arylène éventuellement substitué ou hétéroarylène;
Y est une liaison covalente ou un alkylène dans lequel un atome de carbone peut éventuellement être remplacé par -O-, -S-, ou -NH-, et est éventuellement substitué par hydroxy, alcoxy, amino éventuellement substitué, ou -COR, dans lequel R est hydroxy, alcoxy ou amino;
avec pour condition que quand la substitution éventuelle est hydroxy ou amino ladite substitution ne peut pas être présente sur un atome de carbone adjacent à un hétéroatome; et
Z est hydrogène, aryle monocyclique éventuellement substitué ou hétéroaryle monocyclique éventuellement substitué;
avec pour condition que
(a) Z est hydrogène seulement lorsque Y est une liaison covalente et que X est un 1,4-pyrazolène éventuellement substitué fixé au cycle purine par un atome de carbone; et
(b) lorsque X est un arylène éventuellement substitué, Z est un hétéroaryle monocyclique éventuellement substitué autre que l'imidazole éventuellement substitué,
pour la préparation d'une composition pharmaceutique pour l'accélération de la cicatrisation des blessures chez un mammifère.

2. Utilisation selon la revendication 1, dans laquelle:
R¹ et R² sont indépendamment hydrogène, alkyle en C₁₋₆ éventuellement substitué, ou un groupe -D-E, dans lequel D est une liaison covalente ou un alkylène, et E est un phényle éventuellement substitué, un cycloalkyle éventuellement substitué, un alcényle éventuellement substitué, ou un alcynyle éventuellement substitué, et
R³ est hydrogène.

3. Utilisation selon la revendication 2, dans laquelle:
X est un phénylène éventuellement substitué; et
Y est une liaison covalente ou un alkylène en C₁₋₆ dans lequel un atome de carbone est éventuellement remplacé par -O-, -S-, ou -NH-.

4. Utilisation selon la revendication 3, dans laquelle R¹ et R² sont indépendamment alkyle en C₁₋₆ éventuellement substitué par un cycloalkyle.

5. Utilisation selon la revendication 4, dans laquelle R¹ et R² sont n-propyle, Y est -OCH₂-, et Z est un oxadiazole éventuellement substitué.

6. Utilisation selon la revendication 5, dans laquelle
(i) Z est le 5-(2-méthoxyphényl)-(1,2,4-oxadiazole-3-yle), à savoir la 8-{4-[5-(2-méthoxyphényl)-[1,2,4]oxadiazole-3-ylméthoxy]phényl}-1,3-dipropyl-1,3,7-trihydropurine-2,6-dione;
(ii) Z est le 5-(3-méthoxyphényl)-(1,2,4-oxadiazole-3-yle), à savoir la 8-{4-[5-(3-méthoxyphényl)-[1,2,4]oxadiazole-3-ylméthoxy]phényl}-1,3-dipropyl-1,3,7-trihydropurine-2,6-dione; ou
(iii) Z est le 5-(4-fluorophényl)-(1,2,4-oxadiazole-3-yle), à savoir la 8-{4-[5-(4-fluorophényl)-[1,2,4]oxadiazole-3-ylméthoxy]phényl}-1,3-dipropyl-1,3,7-trihydropurine-2,6-dione.

7. Utilisation selon la revendication 2, dans laquelle:
X est un pyrazolène éventuellement substitué;
Y est une liaison covalente, un alkylène en C₁₋₆ éventuellement substitué par un hydroxy, alcoxy, amino éventuellement substitué, ou -COR, où R est hydroxy, alcoxy ou amino; et
Z est hydrogène, phényle éventuellement substitué, oxadiazolyle éventuellement substitué, isoxazolyle éventuellement substitué, ou pyridyle éventuellement substitué.

8. Utilisation selon la revendication 7, dans laquelle X est le 1,4-pyrazolène éventuellement substitué.

9. Utilisation selon la revendication 8, dans laquelle Z est phényle éventuellement substitué ou pyridyle éventuellement substitué.

10. Utilisation selon la revendication 9, dans laquelle R¹ est un alkyle en C₁₋₆ éventuellement substitué par un cycloalkyle, R² est l'hydrogène, et Y est -CH₂- ou -CH(CH₃)-.

11. Utilisation selon la revendication 10, dans laquelle
(i) R¹ est le n-propyle, X est le 1,4-pyrazolène, Y est -CH₂-, et Z est la 3-trifluorométhylphényle, à savoir la 1-propyl-8-(1-{[3-(trifluorométhyl)-phényl]méthyl}pyrazole-4-yl)-1,3,7-trihydropurine-2,6-dione;
(ii) R¹ est le n-propyle, X est le 1,4-pyrazolène, Y est -CH₂-, et Z est le phényle, à savoir la 1-propyl-8-[1-benzylpyrazole-4-yl]-1,3,7-trihydropurine-2,6-dione;
(iii) R¹ est le n-butyle, X est le 1,4-pyrazolène, Y est -CH₂-, et Z est le 3-fluorophényle, à savoir la 1-butyl-8-(1-{[3-fluorophényl]méthyl} pyrazole-4-yl)-1,3,7-trihydropurine-2,6-dione;
(iv) R¹ est le n-propyle, X est le 1,4-pyrazolène, Y est -CH(CH₃)-, et Z est le phényle, à savoir la 1-propyl-8-[1-(1-phényléthyl)pyrazole-4-yl]-1,3,7-trihydropurine-2,6-dione;
(v) R¹ est le cyclopropylméthyle, X est le 1,4-pyrazolène, Y est -CH₂-, et Z est la 2-pyridyle, à savoir la 1-(cyclopropylméthyl)-8-[1-(2-pyridylméthyl)pyrazole-4-yl]-1,3,7-trihydropurine-2,6-dione; ou
(vi) R¹ est le n-butyle, X est le 1,4-pyrazolène, Y est -CH₂-, et Z est le 6-trifluorométhylpyridine-3-yle, à savoir la 1-n-butyl-8-[1-((6-trifluorométhyl)pyridine-3-ylméthyl)pyrazole-4-yl]-1,3,7-trihydropurine-2,6-dione.

12. Utilisation selon la revendication 9, dans laquelle R¹ et R² sont indépendamment méthyle, éthyle, n-propyle, ou cyclopropylméthyle, et Y est méthylène ou éthylène qui peuvent être éventuellement substitués par hydroxy, alcoxy, amino éventuellement substitué, ou -COR, où R est hydroxy, alcoxy ou amino.

13. Utilisation selon la revendication 12, dans laquelle
(i) R¹ et R² sont le n-propyle, Y est -CH₂-, et Z est le 3-(1,2,3,4-tétrazole-5-yl)phényle, à savoir la 1,3-dipropyl-8-{1-[(3-(1H-1,2,3,4-tétrazole-5-yl)phényl)méthyl]pyrazole-4-yl}-1,3,7-trihydropurine-2,6-dione;
(ii) R¹ est le n-propyle, R² est l'éthyle, Y est -CH₂-, et Z est le 3-trifluorométhylphényle, à savoir la 3-éthyl-1-propyl-8-{1-[(3-trifluorométhylphényl)méthyl]pyrazole-4-yl }-1,3,7-trihydropurine-2,6-dione;
(iii) R¹ et R² sont le n-propyle, Y est -CH(CH₃)-, et Z est le 3-trifluorométhylphényle, à savoir la 1,3-dipropyl-8-(1-{1-[(3-(trifluorométhyl)phényl]éthyl}pyrazole-4-yl)-1,3,7-trihydropurine-2,6-dione;
(iv) R¹ et R² sont le n-propyle, Y est -CH₂-, et Z est le 4-carboxyphényle, à savoir la 1,3-dipropyl-8-{1-[(4-carboxyphényl)méthyl]pyrazole-4-yl}-1,3,7-trihydropurine-2,6-dione;
(v) R¹ et R² sont le n-propyle, Y est -CH₂-, et Z est le 3-carboxyphényle, à savoir l'acide 3-{[4-(2,6-dioxo-1,3-dipropyl-1,3,7-trihydropurine-8-yl)-pyrazolyl]méthyl benzoïque;
(vi) R¹ et R² sont le n-propyle, Y est -CH(CO₂H)-, et Z est le phényle, à savoir l'acide 2-[4-(2,6-dioxo-1,3-dipropyl-1,3,7-trihydropurine-8-yl)pyrazolyl]-2-phénylacétique;
(vii) R¹ est le cyclopropylméthyle, R² est le méthyle, Y est -CH₂-, et Z est le 3-trifluorométhylphényle, à savoir la 1-cyclopropylméthyl-3-méthyl-8-{1-[(3-trifluorométhylphényl)méthyl] pyrazole-4-yl }-1,3,7-trihydropurine-2,6-dione;
(viii) R¹ et R² sont le méthyle, Y est -CH₂-, et Z est le 3-fluorophényle, à savoir la 1,3-diméthyl-8-{1-[(3-fluorophényl)méthyl]pyrazole-4-yl }-1,3,7-trihydropurine-2,6-dione;
(ix) R¹ est le n-propyle, R² est le méthyle, Y est -CH₂-, et Z est le 3-trifluorométhylphényle, à savoir la 3-méthyl-1-propyl-8-{1-[(3-trifluorométhylphényl)méthyl]pyrazole-4-yl}-1,3,7-trihydropurine-2,6-dione;
(x) R¹ et R² sont le n-propyle, Y est -CH₂-, et Z est le 3-(trifluorométhyl)phényle, à savoir la 1,3-dipropyl-8-(1-{[3-(trifluorométhyl)phényl]méthyl}pyrazole-4-yl)-1,3,7-trihydropurine-2,6-dione;
(xi) R¹ et R² sont le n-propyle, Y est -CH₂-, et Z est le 3-fluorophényle, à savoir la 1,3-dipropyl-8-{1-[(3-fluorophényl)méthyl]pyrazole-4-yl}-1,3,7-trihydropurine-2,6-dione;
(xii) R¹ est l'éthyle, R² est le méthyle, Y est -CH₂-, et Z est le 3-fluorophényle, à savoir la 1-éthyl-3-méthyl-8-{1-[(3-fluorophényl)méthyl]pyrazole-4-yl}-1,3,7-trihydropurine-2,6-dione; ou
(xiii) R¹ et R² sont le n-propyle, Y est -CH₂-, et Z est le 2-méthoxyphényle, à savoir la 1,3-dipropyl-8-{1-[(2-méthoxyphényl)méthyl]pyrazole-4-yl}-1,3,7-trihydropurine-2,6-dione.

14. Utilisation selon la revendication 8, dans laquelle Z est un oxadiazole éventuellement substitué.

15. Utilisation selon la revendication 14, dans laquelle R¹ est un alkyle en C₁₋₆ éventuellement substitué par un cycloalkyle, R² est H, et Y est -CH₂- ou -CH(CH₃)-.

16. Utilisation selon la revendication 15, dans laquelle
(i) R¹ est le n-propyle, X est le 1,4-pyrazolène, Y est -CH₂-, et Z est le 5-(4-chlorophényle)-[1,2,4]-oxadiazole-3-yle, à savoir la 8-(1-{[5-(4-chlorophényl)(1,2,4-oxadiazole-3-yl)]méthyl}pyrazole-4-yl)-1-propyl-1,3,7-trihydropurine-2,6-dione; ou
(ii) R¹ est le n-butyle, X est le 1,4-pyrazolène, Y est -CH₂-, et Z est le 5-(4-chlorophényle)-[1,2,4]-oxadiazole-3-yle, à savoir la 8-(1-{[5-(4-chlorophényl)(1,2,4-oxadiazole-3-yl)]méthyl pyrazole-4-yl)-1-butyl-1,3,7-trihydropurine-2,6-dione.

17. Utilisation selon la revendication 14, dans laquelle R¹ et R² sont indépendamment un alkyle en C₁₋₆ éventuellement substitué par un cycloalkyle, et Y est -CH₂- ou -CH(CH₃)-.

18. Utilisation selon la revendication 17, dans laquelle
(i) R¹ et R² sont le n-propyle, Y est -CH₂-, et Z est le 3-(4-chlorophényl)-[1,2,4]-oxadiazole-5-yle, à savoir la 8-(1-{[3-(4-chlorophényl)(1,2,4-oxadiazole-5-yl)]méthyl}pyrazole-4-yl)-1,3-dipropyl-1,3,7-trihydropurine-2,6-dione; ou
(ii) R¹ est le n-propyle, R² est l'éthyle, Y est -CH₂-, et Z est le 3-(4-chlorophényl)-[1,2,4]-oxadiazole-5-yle, à savoir la 8-(1-{[3-(4-chlorophényl)(1,2,4-oxadiazole-5-yl)]méthyl}pyrazole-4-yl)-3-éthyl-1-propyl-1,3,7-trihydropurine-2,6-dione.

19. Utilisation selon la revendication 8, dans laquelle Z est l'hydrogène.

20. Utilisation selon la revendication 19, dans laquelle R¹ et R² sont indépendamment un alkyle en C₁₋₆ éventuellement substitué par un cycloalkyle, et Y est une liaison covalente.

21. Utilisation selon la revendication 20, dans laquelle
(i) R¹ et R² sont le n-propyle, à savoir la 1,3-dipropyl-8-pyrazole-4-yl-1,3,7-trihydropurine-2,6-dione; et
(ii) R² est le sec-butyle, et R¹ est le méthyle, à savoir la 1-méthyl-3-sec-butyl-8-pyrazole-4-yl-1,3,7-trihydropurine-2,6-dione.

22. Utilisation selon la revendication 8, dans laquelle Z est un isoxazolyle éventuellement substitué.

23. Utilisation selon la revendication 22, dans laquelle R¹ et R² sont indépendamment un alkyle en C₁₋₆ éventuellement substitué par un cycloalkyle, et Y est -CH₂-, -CH(CH₃)-, ou une liaison covalente.

24. Utilisation selon la revendication 23, dans laquelle
(i) R¹ et R² sont le n-propyle, Y est -CH₂-, et Z est le 5-(4-trifluorométhylphényl)isoxazole-3-yle, à savoir la 1,3-dipropyl-8-[1-({5-[4-(trifluorométhyl)phényl]isoxazole-3-yl}méthyl)pyrazole-4-yl]-1,3,7-trihydropurine-2,6-dione; ou
(ii) R¹ est le n-propyle, R² est l'éthyle, Y est -CH₂-, et Z est le 5-(4-chlorophényl)isoxazole-3-yle, à savoir la 8-(1-{[5-(4-chlorophényl)isoxazole-3-yl]méthyl}pyrazole-4-yl)-3-éthyl-1-propyl-1,3,7-trihydropurine-2,6-dione.

25. Utilisation selon la revendication 8, dans laquelle Z est un pyridyle éventuellement substitué.

26. Utilisation selon la revendication 25, dans laquelle R¹ et R² sont indépendamment un alkyle enC₁₋₆ éventuellement substitué par un cycloalkyle, et Y est -CH₂-, -CH(CH₃)-, ou une liaison covalente.

27. Utilisation selon la revendication 26, dans laquelle
(i) R¹ et R² sont le n-propyle, Y est -CH₂-, et Z est le pyrid-2-yle, à savoir la 1,3-dipropyl-8-[1-(2-pyridylméthyl)pyrazole-4-yl]-1,3,7-trihydropurine-2,6-dione;
(ii) R¹ et R² sont le n-propyle, Y est -CH₂-, et Z est le 6-trifluorométhylpyrid-3-yle, à savoir la 1,3-dipropyl-8-(1-{[6-(trifluorométhyl)(3-pyridyl)]méthyl}pyrazole-4-yl)-1,3,7-trihydropurine-2,6-dione;
(iii) R¹ et R² sont le n-propyle, Y est -CH₂-, et Z est le 6-carboxy-pyrid-2-yle, à savoir l'acide 6-{[4-(2,6-dioxo-1,3-dipropyl-1,3,7-trihydropurine-8-yl)pyrazolyl]méthyl}pyridine-2-carboxylique;
(iv) R¹ est le n-propyle, R² est l'éthyle, Y est -CH₂-, et Z est le 2-pyridyle, à savoir la 3-éthyl-1-propyl-8-[1-(2-pyridylméthyl)pyrazole-4-yl]-1,3,7-trihydropurine-2,6-dione;
(v) R¹ est le n-propyle, R² est l'éthyle, Y est -CH₂-, et Z est le 6-(trifluorométhyl)-pyrid-3-yle, à savoir la 3-éthyl-1-propyl-8-(1-{[6-(trifluorométhyl)(3-pyridyl)]méthyl}pyrazole-4-yl)-1,3,7-trihydropurine-2,6-dione;
(vi) R¹ est le cyclopropylméthyle, R² est l'éthyle, Y est -CH₂-, et Z est le 6-(trifluorométhyl)-pyrid-3-yle, à savoir la 1-(cyclopropylméthyl)-3-éthyl-8-(1-{[6-(trifluorométhyl)(3-pyridyl)]méthyl } pyrazole-4-yl)-1,3,7-trihydropurine-2,6-dione; ou
(vii) R¹ est le 2-méthylpropyle, R² est l'éthyle, Y est -CH₂-, et Z est le 6-(trifluorométhyl)-pyrid-3-yle, à savoir la 3-éthyl-1-(2-méthylpropyl)-8-(1-{[6-(trifluorométhyl)(3-pyridyl)]méthyl pyrazole-4-yl)-1,3,7-trihydropurine-2,6-dione.

28. Utilisation selon la revendication 1, dans laquelle le mammifère est un humain, ou est un animal domestique.

29. Utilisation selon la revendication 1, dans laquelle la composition pharmaceutique est pour administration topique, administration systémique, ou administration directement sur la blessure.

30. Utilisation selon la revendication 1, dans laquelle la blessure est provoquée par un traumatisme mécanique, chimique ou thermique.

31. Utilisation selon la revendication 30, dans laquelle la blessure est le résultat d'une incision chirurgicale.

32. Utilisation selon la revendication 30, dans laquelle la blessure est choisie dans le groupe consistant en contusions, brûlures, incisions, et lacérations.

33. Utilisation selon la revendication 1, dans laquelle la blessure est associée à une maladie ou un trouble.

34. Utilisation selon la revendication 33, dans laquelle la blessure est un ulcère diabétique.

35. Composition pharmaceutique pour utilisation dans l'augmentation de la cicatrisation des blessures et convenant pour la délivrance topique, comprenant une quantité thérapeutiquement efficace d'un récepteur A_{2B} ayant la structure selon Formule 1 ou Formule II: 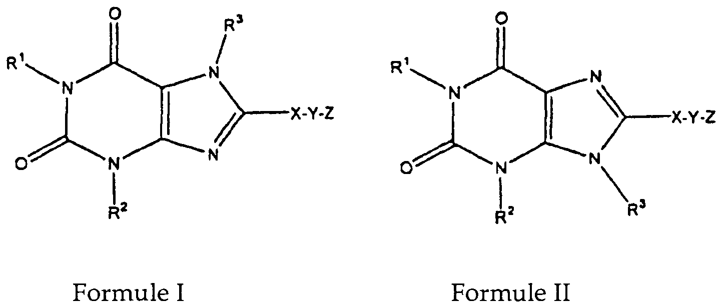 où:
R¹ et R² sont choisis indépendamment parmi hydrogène, alkyle éventuellement substitué, ou un groupe -D-E, dans lequel D est une liaison covalente ou un alkylène, et E est un alcoxy éventuellement substitué, un cycloalkyle éventuellement substitué, un aryle éventuellement substitué, un hétéroaryle éventuellement substitué, un hétérocyclyle éventuellement substitué, un alcényle éventuellement substitué, ou un alcynyle éventuellement substitué, avec pour condition que quand D est une liaison covalente E ne peut pas être alcoxy;
R³ est hydrogène, alkyle éventuellement substitué ou cycloalkyle éventuellement substitué;
X est arylène éventuellement substitué ou hétéroarylène;
Y est une liaison covalente ou un alkylène dans lequel un atome de carbone peut éventuellement être remplacé par -O-, -5-, ou -NH-, et est éventuellement substitué par hydroxy, alcoxy, amino éventuellement substitué, ou -COR, dans lequel R est hydroxy, alcoxy ou amino;
avec pour condition que quand la substitution éventuelle est hydroxy ou amino ladite substitution ne peut pas être présente sur un atome de carbone adjacent à un hétéroatome; et
Z est hydrogène, aryle monocyclique éventuellement substitué ou hétéroaryle monocyclique éventuellement substitué;
avec pour condition que
(a) Z est hydrogène seulement lorsque Y est une liaison covalente et que X est un 1,4-pyrazolène éventuellement substitué fixé au cycle purine par un atome de carbone; et
(b) lorsque X est un arylène éventuellement substitué, Z est un hétéroaryle monocyclique éventuellement substitué autre que l'imidazole éventuellement substitué, et
un support pharmaceutiquement acceptable.

36. Composition pharmaceutique pour utilisation dans l'augmentation de la cicatrisation des blessures et convenant pour la délivrance topique selon la revendication 35, qui est un onguent, une crème ou un gel.
